# EUROPEAN PATENT APPLICATION

(11) **EP 4 374 848 A1**
(43) Date of publication of application: **29.05.2024**
(21) Application number: 22845427.8
(22) Date of filing: 22.07.2022
(51) Int. Cl.: A61K 9/00, A61K 45/06, A61K 38/46

(54) **ANTIBIOTIC PHARMACEUTICAL COMPOSITION CAPABLE OF SUBCUTANEOUS ADMINISTRATION**

(30) Priority: 23.07.2021 CN 202110838181
(71) Applicant: Shanghai Bao Pharmaceuticals Co., Ltd., Shanghai 201908 (CN)
(72) Inventor: LIU, Yanjun, Shanghai 215123 (CN); WANG, Zheng, Shanghai 215123 (CN); LU, Lin, Shanghai 215123 (CN); ZHU, Zhen, Shanghai 215123 (CN)
(74) Representative: Bayramoglu et al.
(86) International application number: PCT/CN2022/107252
(87) International publication number: WO 2023/001264

(57) **Abstract**

The present invention discloses an antibiotic pharmaceutical composition for intradermal or subcutaneous administration, which comprises an antibiotic and hyaluronidase (HAase). The present invention further relates to a kit comprising the pharmaceutical composition, a method for preparing the kit, and use of the pharmaceutical composition and the kit.

## Description

### Technical Field

The present invention relates to the technical field of pharmaceutical preparations, and particularly to an antibiotic pharmaceutical composition suitable for intradermal or subcutaneous administration, a kit comprising the composition and a preparation method thereof, and use of the composition and the kit.

### Background

Antibiotics are the most commonly used drugs against infections in clinic. After many years of development, thousands of kinds of antibiotics are available, and hundreds of them commonly used in clinic, mainly including β-lactams, aminoglycosides, macrolides, lincomycin, polypeptides, quinolones, sulfanilamides, anti-tuberculosis agents, antifungal agents and other antibiotics.

Antibiotics can be administered through many routes, among which oral administration, intramuscular injection and intravenous administration are common. However, antibiotics administered through these routes have their own limitations, shortcomings and side effects.

Intravenous administration refers to a route of administration in which a drug solution is allowed to directly enter the body by intravenous infusion or bolus injection. This route has the advantages of fast onset of action and high bioavailability and is a commonly used route of administration in clinic. However, it is also recognized as the most dangerous route of administration. In addition to the risk of infection caused by deep intervention, insoluble particles are inevitably introduced in the process of drug production, transportation, storage and formulation, causing adverse reactions such as vascular embolism, phlebitis, heat-like reactions and granuloma. In addition, some antibiotics are unstable in the gastrointestinal tract and tend to be damaged by the gastrointestinal tract, so they can only be injected intravenously. Moreover, the operation during intravenous administration is technically difficult, and usually necessitates professional medical staff in medical institutions, which brings inconvenience to the treatment of patients and greatly occupies medical resources. Intravenous administration requires venipuncture and needle placement. For some patients with poor blood vessel status, such as the elderly, infants and severely burned patients, venipuncture is difficult.

Oral administration is the most commonly used mode of administration in drug therapy, which has the advantages of convenient administration, no pain, simple operation, low administration cost, safety over intravenous administration, and less adverse reactions related to injection. At present, some studies show that although oral antibiotics have such advantages, their disadvantages are becoming increasingly notable. They have irritations on the gastrointestinal tract or adverse effects on the digestive system, and also studies show that oral antibiotics affect the growth of infants. At present, exposure to oral antibiotics has been confirmed to interfere with intestinal microbial colonization, leading to the risks of increased incidence of asthma and diabetes type 1. Also, studies show that the use of oral antibiotics during or before patients with tumors receive immunotherapy causes intestinal flora disturbance, which will seriously reduce the efficacy of immunotherapy (Routy, B et al. (2018). Science 359(6371): 91-97).

Intramuscular injection is rarely used as a route of administration for antibiotics because of its disadvantages, such as a low dose volume of 5 mL or less, pain and discomfort in patients, slow onset of action, and nerve damage caused by improper injection.

Intradermal or subcutaneous administration refers to a route in which a drug is injected into an intradermal or subcutaneous tissue, so that the drug can be absorbed quickly. Compared with intravenous administration, subcutaneous administration has the advantages of lower cost, more sites available, easier needle insertion operation, less pain or discomfort than intravenous needle insertion, and easier change of the needle insertion site than in intravenous needle insertion, and ability to position the needle at a relatively insensitive site on the skin. Moreover, a drug solution can be dosed by intradermal or subcutaneous administration in almost any environment. Intradermal or subcutaneous injection is more suitable than intravenous infusion in the case of lack of nursing conditions. However, when the volume of intradermal or subcutaneous injection exceeds 2 mL, tissue deformation is caused, thus increasing the stress between tissues, causing pain and discomfort, and limiting the volume and speed of subcutaneous injection at a single site. Due to the limited volume of injection and the potential irritation of high-concentration antibiotics to the skin, antibiotics are mainly administered intravenously and orally in clinic.

Therefore, there is an urgent need for an antibiotic for subcutaneous administration, which is safe, acts quickly and will not cause discomfort to patients.

### Summary of the Invention

In view of the defects in the prior art, the present invention aims to provide an pharmaceutical composition for intradermal or subcutaneous administration. The antibiotic pharmaceutical composition comprises an antibiotic and hyaluronidase (HAase). The pharmaceutical composition has obvious advantages over antibiotics for oral administration, intramuscular injection and intravenous administration. For example, the blood drug concentration (Cₘₐₓ) of the antibiotic in the composition is increased, compared with that in the case where the antibiotic is administered alone; the time (Tₘₐₓ) required to reach the highest blood drug concentration is reduced, compared with that in the case where the antibiotic is administered alone; and/or when the composition is subcutaneously administered, the reductions in the number and diversity of intestinal microbial flora relative to those before administration are significantly decreased, compared with the reductions in the case where the antibiotic is orally administered.

Specifically, the present invention relates to the following items:
1. A pharmaceutical composition for intradermal or subcutaneous administration, comprising an antibiotic and hyaluronidase (HAase).
2. The pharmaceutical composition according to Item 1, where the content of the antibiotic is 10 mg/mL-5 g/mL; and the content of the HAase is 45 units/ml-4500000 units/ml.
3. The pharmaceutical composition according to Item 1 or 2, where the antibiotic is selected from the group consisting of β-lactams, aminoglycosides, macrolides, lincomycins, polypeptides, tetracyclines, quinolones, sulfanilamides, anti-tuberculosis agents, and antifungal agents.
4. The pharmaceutical composition according to Item 3, where the β-lactam antibiotics are selected from the group consisting of Penicillin G, Penicillin V, Pheneticillin, Oxacillin, Methicillin, Cloxacillin, Dicloxacillin, Ampicillin, Amoxicillin, Pivampicillin, Carbenicillin, Piperacillin, Sulbenicillin, Temocillin, Mezlocillin, Mecillinam, Amdinocillin Pivoxil, Apalcillin, Aspoxicillin, Azidocillin, Azlocillin, Bacampicillin, Benzylpenicillin, Benzylpenicillin sodium, Carindacillin, Clometocillin, Ciclacillin, Epicillin, Fenbenicillin, Flucloxacillin, Hetacillin, Lenampicillin, Metampicillin, Methicillin sodium, Nafcillin, Penamecillin, Penethamate hydriodide, Penicillin G Benethamine, Benzathine Penicillin G, Penicillin G benzhydrylamine, Penicillin G calcium, Penicillin G hydrabamine, Penicillin G potassium, Penicillin G procaine, Penicillin N, Penicillin O, Penicillin V Benzathine, Penicillin V hydrabamine, Penimepicycline, Pheneticillin potassium, Propicillin, Quinacillin, Sulbenicillin, Sultamicillin, Talampicillin, Tazocillin, TicarcillinLoracarbef, 3-chloro-1-Carbacephem, 3-thiocarbacephem, Flomoxef, Latamoxef, Latamoxef, Cefazolin, Cefalexin, Cefalotin, Cefradine, Ceftezole, Cefuroxime, Cefaclor, Cefamandole, Cefotiam, Cefonicid, Ceforanide, Cefoperazone, Ceftriaxone, Ceftazidime, Cefotaxime, Ceftizoxime, Cefixime, Cefodizime, Cefpiramide, Cefpirome, Cefepime, Cefclidin, Cefadroxil, Cefatrizine, Cefazedone, Cefcapene pivoxil, Cefolidin, Cefdinir, Cefditoren, Cefetamet, Cefmenoxime, Cefotetan, Cefozopran, Cefpimizole, Cefpodoxime Proxetil, Cefprozil, Cefroxadine, Cefsulodin, Cefteram, Ceftibuten, Ceftizoxime, Cefuzonam, Cephatril, Cefaloglycin, Cefaloridine, Cephalosporin, Cephalotin, Cephapirin, Cefbuperazone, Cefminox, Imipenem, Meropenem, Panipenem, Biapenem, Ertapenem, Faropenem, Cefoxitin, Cefmetazole, Aztreonam, Carumonam, Latamoxef, Flomoxef, clavulanic acid, clavulanate, clavulanate, Sulbactam, and Tazobactam;
   the aminoglycoside antibiotics are selected from the group consisting of Streptomycin, Kanamycin, Gentamicin, Amikacin, Tobramycin, Netilmicin, Sisomicin, Albomycin, Arbekacin, Bambermycin, Butyrosin, Dibekacin, Dihydrostreptomycin, Fortimicin, Isepamicin, Micronomicin, Neomycin, Neodecyllin, Paromomycin, Ribostamycin, Spectinomycin, and Trospectomycin;
   the macrolide antibiotics are selected from the group consisting of Erythromycin, Clarithromycin, Azithromycin, Roxithromycin, Carbomycin, Dirithromycin, Erythromycin Acistrate, Erythromycin Estolate, Erythromycin Gluceptate, Erythromycin Lactobionate, Erythromycin Stinoprate, Erythromycin stearate, Josamycin, Leucomycin, Midecamycin, Miokamycin, Oleandomycin, Primycin, Rokitamycin, Rosaramicin, Spiramycin, and Troleandomycin;
   the lincomycin antibiotics are selected from the group consisting of Lincomycin and Clindamycin;
   the polypeptide antibiotics are selected from the group consisting of Polymixin B, Polymixin E, Norvancomycin, Teicomycin, Vancomycin, Teicoplanin, Amphomycin, Bacitracin, Capreomycin, Colistin, Colomycin, Enduracidin, Enviomycin, Fusafungine, Gramicidin, Mikamycin, Polymixin, Pristinamycin, Dalfopristin, Ristocetin, Thiostrepton, Tuberactinomycin, Tyrocidine, Tyrothricina, Viomycin, Virginiamycin, and Zinc Bacitracin;
   the tetracycline antibiotics are selected from the group consisting of Apicycline, Chlortetracycline, Clomocycline, Demeclocycline, Doxycycline, Guamecycline, Lymecycline, Meclocycline, Metacycline, Minocycline, Oxytetracycline, Penimepicycline, Pipacycline, Rolitetracycline, Sancycline, Tetracycline, Cycloserine, Mupirocin, and Tuberin;
   the quinolone antibiotics are selected from the group consisting of Nalidixic Acid, Pipemidic Acid, Norfloxacin, Ofloxacin, Ciprofloxacin, Enoxacin, Pefloxacin, Levofloxacin, Gatifloxacin, Moxifloxacin, Norfloxacin, Fleroxacin, Lomefloxacin, Sparfloxacin, Grepafloxacin, Rufloxacin, Clinafloxacin, Balofloxacin, Trovafloxacin, Fluoroquinolone, Alatrofloxacin Mesylate, Cinoxacin, Difloxacin, Flumequine, Grepafloxacin, Miloxacin, Marbofloxacin, Nadifloxacin, Oxolinic acid, Pazufloxacin, Piromidic acid, Rosoxacin, Temafloxacin, Tosufloxacin, and Trovafloxacin mesilate;
   the sulfanilamide antibiotics are selected from the group consisting of Sulfamethoxazole, Trimethoprim, acetyl Sulfamethoxypyridazine, Benzylsulfamide, Chloramine B, Chloramine T, Dichloramine T, Sulfisomidine, β-GlucosylSulfanilamide, Mafenide, 4'-methylamino sulfonyl-sulfonanilide, Noprylsulfamide, Phthalylsulfacetamide, Phthalylsulfathiazole, Salicylazosulfapyridine, Sulfasuxidine, Sulfabenzamide, Sulfacetamide, Sulfachlorpyridazine, Sulfachrysoidine, Sulfacitine, Sulfadiazine, Sulfadicramide, Sulfadimethoxine, Sulfadoxine, Sulfaethidole, Sulfaguanidine, Sulfaguanole, Sulfalene, Sulfaloxic Acid, Sulfamerazine, Oxymethylpyrimidine, Sulfamerazine, Sulfamethizole, Sulfametomidine, Sulfisoxazole, Sulfamethoxypyridazine, Sulfametrole, Sulfamidochrysoidine, Sulfazole, Sulfanilamide, 4-Sulfanilamidosalicylic acid, Sulfanilamidosulfanilamide, Sulfanilylurea, n-Sulfanilamido-3,4-dicarboxamide, Sulfanitran, Sulfaperin, Sulfaphenazole, Sulfaproxyline, Sulfapyrazole, Sulfapyridine, Sulfasomizole, Sulfasymazine, Sulfathiazole, Sulfathiourea, Sulfatolamide, Sulfisomidine, and Sulfatroxazole;
   the furan antibiotics are selected from the group consisting of Nitrofurantoin, Furazolidone, Furaltadone, Furazolium Chloride, Nifuradene, Nifuratel, Nifurfoline, Nifurpirinol, Nifurprazine, and Nifurtoinol;
   the nitroimidazole antibiotics are selected from the group consisting of Metronidazole, Tinidazole and Omidazole;
   the anti-tuberculosis antibiotics are selected from the group consisting of Isoniazid, Rifampicin, Rifamide, Pyrazinamide and Ethambutol; and/or
   the antifungal antibiotics are selected from the group consisting of amphotericin B, fluconazole, Itraconazole, 5-Flucytosine, Candicidin, Dermostatin, Filipin, antifungal pigments, Hachimycin, Hamycin, Lucensomycin, Mepartricin, Pimaricin, Natamycin, Nysfungin, Pecilocin, Permycin, Azaserine, Griseofulvin, Oligomycin, Neodecyllin, Pyrrolnitrin, Siccanin, Tubercidin, Viridin, Allyl amine, Butenafine, Naftifine, Terbinafine, imidazole, Bifonazole, Butoconazole, Chlordantoin, Chlormidazole, Cloconazole, Clotrimazole, Econazole, Enilconazole, Fenticonazole, Flutrimazole, Isoconazole, Ketoconazole, Lanoconazole, Miconazole, Omoconazole, Oxiconazole nitrate, Sertaconazole, Sulconazole, Tioconazole, Voriconazole, thiocarbamate, Tolciclate, Tolindate, Tolnaftate, triazole, Saperconazole, Terconazole, Acrisorcin, Amorolfine, Xenysalate, Bromosalicylchloranilide, Buclosamide, Calcium propionate, Chlorphenesin, Coparaffinate, Dimazole dihydrochloride, Exalamide, Flucytosine, Haletazole, Hexetidine, lipopeptide such as Echinocandin, Loflucarban, Nifuratel, Potassium iodide, Propanoic acid, 2-Mercaptopyridine oxide, Salicylanilide, Sodium propionate, Sulbentine, Tenonitrozole, Triacetin, Benzthiadiazine acetate, Undecenoic acid, and Zinc propionate.
5. The pharmaceutical composition according to any one of Items 1 to 4, where the HAase has the activity of degrading hyaluronic acid under neutral conditions; preferably, the HAase is selected from the group consisting of HAase extracted from animal testis, recombinant animal HAase or a mutant thereof, recombinant and/or extracted bacterial HAase, recombinant human HAase or a mutant thereof; preferably, the HAase is recombinant human HAase or a mutant thereof; further preferably, the HAase is recombinant human HAase or a mutant thereof; more preferably, the recombinant human HAase comprises an amino acid sequence as shown in SEQ ID NO. 1; and most preferably, the recombinant human HAase comprises an amino acid sequence as shown in SEQ ID NO. 2.
6. The pharmaceutical composition according to any one of Items 1 to 5, where the HAase has an enzyme activity of 45 units/ml-3000000 units/ml, preferably 45 units/ml-1500000 units/ml, more preferably 50 units/ml-30000 units/ml, and most preferably 100 units/ml-3000 units/ml.
7. The pharmaceutical composition according to any one of Items 1 to 6, where the Cₘₐₓ of the antibiotic in the composition is increased by at least 10%, for example, at least 20%, at least 30%, at least 40%, and at least 50%, compared with that in the case where the antibiotic is administered alone; and/or the Tₘₐₓ of the antibiotic in the composition is reduced by at least 20%, for example, at least 30%, at least 40%, and at least 50%, compared with that in the case where the antibiotic is administered alone; and/or the AUCₗₐₛₜ of the antibiotic in the composition is increased by at least 5%, or at least 10%, compared with that in the case where the antibiotic is administered alone.
8. The pharmaceutical composition according to any one of Items 1 to 7, where when the composition is subcutaneously administered, the reduction in the diversity of intestinal microbial flora relative to that before administration is 90% or less, for example, 80% or less, 70% or less, 60% or less, and 50% or less, of the reduction relative to the diversity before administration when the antibiotic is administered orally.
9. The pharmaceutical composition according to any one of Items 1 to 8, optionally comprising a pharmaceutically acceptable adjuvant.
10. The pharmaceutical composition according to Item 9, where the pharmaceutically acceptable adjuvant is selected from the group consisting of a buffer, a stabilizer, a nonionic surfactant, a co-solvent, a preservative and/or an excipient.
11. The pharmaceutical composition according to Item 10, where the buffer is selected from the group consisting of a histidine buffer, an acetic acid buffer, a phosphate buffer, a citric acid buffer, and Tris buffer, preferably, a phosphate buffer, and most preferably disodium hydrogen phosphate;
   the stabilizer is selected from the group consisting of trehalose, sucrose, mannitol, sodium chloride, methionine, and disodium edetate, preferably trehalose, mannitol, methionine, and sucrose, and most preferably, a combination of methionine and trehalose;
   the nonionic surfactant is selected from the group consisting of polysorbate 20, polysorbate 80, and poloxamer 188, preferably polysorbate 20 and polysorbate 80, and most preferably polysorbate 20;
   the co-solvent is selected from the group consisting of sodium carbonate, phosphoric acid, citric acid, sodium bicarbonate, sodium hydroxide, sodium chloride, and L-arginine, and preferably sodium bicarbonate and sodium hydroxide;
   the preserver is selected from the group consisting of glycerol, methyl paraben, propyl paraben, benzoic acid, sodium benzoate, and ethanol; and/or
   the excipient is selected from the group consisting of sorbitol, mannitol, trehalose, glycerol, lactose, sucrose, trehalose, maltose and glucose, preferably mannitol, trehalose and sucrose, and most preferably a combination of trehalose and mannitol.
12. The pharmaceutical composition according to Item 11, where
   the concentration of the buffer is 1-100 mM, and preferably 5-50 mM, for example, 5 mM, 10 mM or 50 mM;
   the concentration of the stabilizer is 1-500 mM, and preferably 30-150 mM, for example, 5mM methionine+25mM trehalose, 5mM methionine+53mM trehalose, 5mM methionine+100mM trehalose, lOmM methionine+25mM trehalose, 10mM methionine+53mM trehalose, 10mM methionine+100mM trehalose, 50mM methionine+25mM trehalose, 50mM methionine+53mM trehalose, and 50 mM methionine+100mM trehalose;
   the concentration of the excipient is 1-500 mM, and preferably 160-280 mM, for example, 160 mM, 220 mM or 280 mM;
   the concentration of the surfactant is 0.01-0.1% (w/v), preferably 0.02-0.04% (w/v), and most preferably 0.02% (w/v), and/or
   the concentration of the co-solvent is 0.01 g/L-100 g/L.
13. The pharmaceutical composition according to any one of Items 1 to 12, which is a lyophilized preparation and/or a liquid preparation.
14. A kit, comprising the pharmaceutical composition according to any one of Items 1 to 13, wherein the antibiotic and the HAase in the kit are packaged in mixture or separately.
15. The kit according to Item 14, wherein the separately packaged antibiotic and/or the separately packaged HAase are lyophilized preparations or liquid preparations.
16. The kit according to Item 15, wherein the lyophilized preparation is reconstituted before administration to a subject, and the liquid preparation is directly administered to the subject or diluted before administration to the subject.
17. The kit according to any one of Items 14 to 16, wherein the antibiotic and the HAase are administered sequentially or simultaneously, and the separately packaged antibiotic and/or the separately packaged HAase are administered sequentially or simultaneously.
18. The kit according to Item 17, wherein the antibiotic and the HAase are administered sequentially through a three-way infusion tube, and the administration speed is preferably controlled by manually pushing, by an infusion pump, or by gravity.
19. A method for preparing the kit according to any one of Items 15 to 18, comprising:
   (a) providing an antibiotic;
   (b) providing hyaluronidase (HAase); and
   (c) optionally providing a pharmaceutically acceptable adjuvant,
   wherein the antibiotic and the HAase are respectively prepared into a lyophilized preparation or a liquid preparation; or the antibiotic and the HAase are mixed and then prepared into a lyophilized preparation or a liquid preparation.
20. An injection system, selected from the group consisting of a syringe, an infusion pump, an injection pen, a needleless device, and other delivery devices, wherein the injection system is filled with the pharmaceutical composition according to any one of Items 1 to 14.
21. Use of the pharmaceutical composition according to any one of Items 1 to 14 and the kit according to any one of Items 15 to 18 in the preparation of drugs for treating diseases selected from the group consisting of infections with bacteria, fungi, actinomycetes, mycoplasma, chlamydia, spirochete, and amoeba and diseases caused by the infections.
22. The use according to Item 21, wherein the bacteria are selected from the group consisting of Escherichia, Pseudomonas, Klebsiella, Acinetobacter, Enterobacter, Citrobacter, Haemophilus, Proteus, Salmonella, Shigella, Serratia, Corynebacterium, Staphylococcus, Streptococcus, Enterococcus, Neisseria, Mycobacterium, and Legionella; and
   the fungi are selected from the group consisting of Trichophyton, Epidermophyton, Microsporum, Candida, Cryptococcus, Coccidioides, Histoplasma, Sporothrix, Blastomyces, Geotrichum, Aspergillus, Mucor and Penicillium.
23. The use according to Item 21 or 22, wherein the bacteria or fungi are selected from the group consisting of Haemophilus influenzae type B, Pseudomonas aeruginosa types A and B, Staphylococcus aureus, Streptococcus group B, Streptococcus pneumoniae types (1, 3, 4, 6, 7, 8, 9, 12, 14, 18, 19 and 23), Staphylococcus epidermidis, Staphylococcus saprophyticus, Streptococcus pyogenes, Streptococcus viridans, Streptococcus agalactiae, Diplococcus pneumoniae, Bacillus anthracis, Corynebacterium diphtheriae, Bordetella pertussis, Bacillus tetani, Mycobacterium tuberculosis, Bacillus, Clostridium perfringens, Mycobacterium leprae, Gonococcus, Meningococcus, Legionella pneumophila, Shigella dysenteriae, Pseudomonas aeruginosa, Bacillus proteus, Vibrio cholerae, Vibrio parahaemolyticus, Escherichia coli, Salmonella typhi, Salmonella paratyphi, Salmonella, Haemophilus influenzae, Acinetobacter, Aspergillus, Candida, Cryptococcus, Mucor or Fusarium.
24. The use according to any one of Items 21 to 23, wherein the disease is chronic osteomyelitis caused by Staphylococcus aureus, endocarditis caused by drug-resistant Staphylococcus aureus or Enterococcus, typhoid fever, paratyphoid fever, bacterial food poisoning, bacterial infectious diarrhea, cholera, bacillary dysentery, brucellosis, plague, anthrax, diphtheria, pertussis, scarlatina, epidemic cerebrospinal meningitis, tuberculosis, bacterial blood infection, bacterial upper respiratory tract infection, bacterial lower respiratory tract infection, bacterial urinary tract infection, bacterial abdominal cavity infection, dermatophytosis, mycotic stomatitis, candida vaginitis, fungal pneumonia, fungal urinary tract infection, fungemia, cryptococcosis, candidiasis, aspergillosis, and pneumocystis.

### Detailed Description

The present invention provides an antibiotic for subcutaneous administration. Upon administration, the antibiotic for subcutaneous administration in the present invention is not limited by the volume, acts quickly, has high blood drug concentration and high patient comfort, and will not cause gastrointestinal microbial disturbance.

### 1. Definitions

All technical and scientific terms used herein have the same meaning as understood by those skilled in the art to which the present invention belongs, unless otherwise defined.

"Combined with" means that two or more therapeutic agents are administered to a subject as a mixture, simultaneously as single agents or sequentially as single agents in any order.

"Pharmaceutical composition" refers to a product obtained by mixing an antibiotic with HAase, and includes both fixed and non-fixed combinations. The pharmaceutical composition usually contains a pharmaceutically acceptable adjuvant. The "fixed combination" refers to a single pharmaceutical composition comprising the antibiotic and HAase that are administered simultaneously in a single entity or dosage form. The "non-fixed combination" refers to separate pharmaceutical compositions or unit dosage forms of the antibiotic and HAase, which are administered simultaneously, concurrently or sequentially as independent entities with no restrictions by specific time interval, wherein such administration provides effective levels of the two compounds in a subject.

"Antibiotics" refers to a large class of commonly used anti-infective agents that inhibit and wipe out pathogenic microorganisms such as bacteria and fungi. After many years of development, thousands of kinds of antibiotics are available, and hundreds of them commonly used in clinic, mainly including β-lactams, aminoglycosides, macrolides, lincomycin, polypeptides, quinolones, sulfanilamides, anti-tuberculosis agents, antifungal agents and other antibiotics.

"Hyaluronic acid (HA), also known as hyaluronan, is a linear high-molecular-weight glycosaminoglycan composed of repeatedly connected disaccharide units D-glucuronic acid and N-glucosamine units. It is widely found in connective tissues, mucous tissues, lens of eye and skin in vertebrates, and particularly abundant in embryos, cartilage, synovial fluid, vitreous body, umbilical cord, rooster comb, and other tissues. HA is the most widely distributed acidic mucopolysaccharide in human tissue matrices, which is filled between the collagen fiber structures in the extracellular matrix by forming a network barrier in the body. and thus limits the subcutaneous diffusion of drugs, reduces the speed of drugs reaching blood vessels, and limits the volume of liquid injected subcutaneously The HA barrier is also one of the most important factors that hinder the rapid absorption of subcutaneously injected drug solution.

"HAase" is an endoglycosidase that can degrade hyaluronic acid to produce an effect of reducing the molecular weight. By hydrolyzing the β-1, 4 glycosidic bond in HA chain, small molecular HA or oligosaccharide is obtained, thus increasing the permeability of the tissue and improving the penetration of a liquid through the tissue. HAase, as a "drug diffusion promoting agent", has been used in the medical field for many years, which can promote the diffusion of locally accumulated drug solution, exudate or blood, accelerate the drug absorption, relieve the local tissue tension and pain, and is beneficial to the absorption and dissipation of edema and inflammatory exudate. HAase can be clinically used as a drug penetration enhancer, an anesthetic adjuvant, and a postoperative decongestant, etc.

"Activity" refers to functional activity or activity of a polypeptide or a part thereof related to a full-length (complete) protein. Functional activities include, but are not limited to, biological activities, catalytic or enzymatic activities, antigenicity (ability to bind or compete with polypeptides to bind anti-polypeptide antibodies), immunogenicity, ability to form polymers, and specific binding to receptors or ligands of polypeptides.

"Recombination" in this patent mainly refers to recombinant protein. The recombinant protein is produced by using the technology of recombinant DNA or recombinant RNA to obtain a protein. It can be obtained through an in-vitro or in-vivo method. The principle of the two methods is to use gene recombination technology to obtain a recombinant vector connected with a gene fragment that can be translated into a target protein, and then transfer the recombinant vector into a host cell that can express the target protein to express a specific recombinant protein molecule.

"Mutation" means to change the amino acid sequence of a protein by artificial adjustment, so as to change the function or performance of the protein.

"HAase activity" refers to the ability of HAase to cleave hyaluronic acid. In-vitro assays for determining the activity of HAase (e.g., recombinant human HAase Seph 20) are known in the art and described herein. Exemplary assays include micro-turbidimetric assay described below, which indirectly measures the cleavage of hyaluronic acid by HAase by detecting an insoluble precipitate formed when the uncleaved hyaluronic acid binds to serum albumin.

"Tₘₐₓ" refers to the time when the blood drug concentration reaches a peak after a single administration of a drug.

"Cₘₐₓ" refers to the maximum blood drug concentration on the blood drug concentration-time curve, that is, the highest plasma drug concentration that can be achieved after administration. The peak concentration of a drug is closely related to the clinical use of the drug. A drug is effective only when the peak concentration of the drug reaches the effective concentration, and shows a toxic reaction when the peak concentration is higher than the safe range. In addition, the peak concentration of a drug is also an important index to measure the absorption and safety of a preparation.

"Intestinal microorganisms" refers to all microorganisms colonized in the gastrointestinal tract, including bacteria, fungi and viruses. Of the 29 known bacterial phylum, the Cladosporium and Bacteroides play an important role in the intestines of healthy people, followed by Proteobacteria and Actinobacteria. The intestinal microorganisms are mutually restricted and interdependent, form a symbiotic ecological balance with their hosts, and play an important role in maintaining the intestinal mucosal integrity, metabolism, immune regulation and nutrition. Intestinal flora is closely related to the human health. Studies have found that flora imbalance is related to the occurrence and development of inflammatory bowel disease, colon cancer, obesity and asthma.

The "diversity of intestinal microbial flora" refers to the abundance of species of microbial flora in animal intestines. "The number of intestinal flora" refers to the number of microorganisms such as various bacteria in the intestinal tract of a sample. The commonly used techniques to study the diversity and number of intestinal microbial flora include metagenome sequencing, 16S rRNA sequencing, metatranscriptomic technology, chip technology, fluorescence quantitative PCR and so on. The metagenomic technology can detect the genomes of all microorganisms, including bacteria, fungi and viruses, and is the most comprehensive method for detection. 16S rRNA sequencing can detect bacteria to the level of genus, evaluate the diversity of intestinal bacteria, comprehensively measure the relative content of intestinal bacteria, and predict the metabolic network of genes by software. The macrotranscriptomic technology can quantitatively detect all kinds of intestinal microorganisms (bacteria, viruses, phages, archaea, fungi, yeasts, and parasites) to the level of "species" and even to the level of "strains". Chip technology can qualitatively detect bacteria, fungi and viruses.

"Pharmaceutically acceptable adjuvant" refers to an ingredient other than the active ingredient in a pharmaceutical composition, which is non-toxic to the subject. The pharmaceutically acceptable excipients include, but are not limited to, buffers, excipients, stabilizers, preservatives, pH regulators, nonionic surfactants and/or co-solvents.

"Oral administration" is the most commonly used mode of administration in drug therapy, which has the advantages of convenient administration, no pain, simple operation, low administration cost, safety over intravenous administration, and less adverse reactions related to injection. The disadvantage of oral administration is that the absorption of drugs after oral administration is slow and irregular, and the drug utilization rate is generally low. Some drugs are easily destroyed in the gastrointestinal tract, and thus oral administration is not suitable. In addition, the oral administration of certain drugs may cause irritation to the gastrointestinal tract or adversely affect the digestive system.

"Subcutaneous administration" refers to the injection of drugs into a subcutaneous tissue so that the drugs are absorbed. Subcutaneous administration has the following advantages in economy and operability. 1. Low cost. 2. More sites available, easier needle insertion operation, less pain or discomfort than in intravenous needle insertion, and easier change of the needle insertion site than in intravenous needle insertion, and ability to position the needle at a relatively insensitive site on the skin. 3. Subcutaneous injection can be carried out in almost any environment, and subcutaneous injection is more suitable than intravenous infusion in the case of lack of nursing conditions. Considering the safety, because there is a fibrous skeleton with a fixed structure in the subcutaneous tissue, the flow of molecules with a diameter greater than 200 nm to pass through the subcutaneous tissue is restricted, thus avoiding the risk of introducing large insoluble particles into the central circulation. Subcutaneous injection has the following advantages in safety: 1. There is no risk of thrombosis. 2. It is unlikely to cause pulmonary edema and excessive volume of injection. 3. It will not cause thrombophlebitis. 4. It will not cause complications such as sepsis or systemic infection.

"About" means that the value is within an acceptable error range of a specific value determined by a person of ordinary skill in the art, and the value partly depends on how to measure or determine it, i.e. the limit of the measurement system. In the context of a particular measurement, result or embodiment, "about" means that the value is within one standard deviation according to the practice in the art, or up to 5% (whichever is greater), unless explicitly stated otherwise in the examples or elsewhere in the specification.

### 2. Pharmaceutical composition of the present invention

In a first aspect, the present invention provides an antibiotic pharmaceutical composition for subcutaneous administration, which comprises an antibiotic and HAase. The content of the antibiotic is 10 mg/mL-5 g/mL. The concentration of HAase is 45 units/ml-4500000 units/ml, preferably 45 units/ml-3000000 units/ml, further preferably 45 units/ml-1500000 units/ml, more preferably 50 units/ml-30000 units/ml, and most preferably 100 units/ml-3000 units/ml.

The antibiotic pharmaceutical composition comprises one or more antibiotics and/or recombinant human HAase, as well as a pharmaceutically acceptable adjuvant.

The antibiotics include β-lactams, aminoglycosides, macrolides, lincomycins, polypeptides, tetracyclines, quinolones, sulfanilamides, anti-tuberculosis agents, and antifungal agents.

The β-lactam antibiotics are preferably Penicillin G, Penicillin V, Pheneticillin, oxacillin, Methicillin, Cloxacillin, Dicloxacillin, Ampicillin, Amoxicillin, Pivampicillin, Carbenicillin, Piperacillin, Sulbenicillin, Temocillin, Mezlocillin, Mecillinam, Amdinocillin Pivoxil, Apalcillin, Aspoxicillin, Azidocillin, Azlocillin, Bacampicillin, Benzylpenicillin, Benzylpenicillin sodium, Carindacillin, Clometocillin, Ciclacillin, Epicillin, Fenbenicillin, Flucloxacillin, Hetacillin, Lenampicillin, Metampicillin, Methicillin sodium, Nafcillin, Penamecillin, Penethamate hydriodide, Penicillin G Benethamine, Benzathine Penicillin G, Penicillin G benzhydrylamine, Penicillin G calcium, Penicillin G hydrabamine, Penicillin G potassium, Penicillin G procaine, Penicillin N, Penicillin O, Penicillin V Benzathine, Penicillin V hydrabamine, Penimepicycline, Pheneticillin potassium, Propicillin, Quinacillin, Sulbenicillin, Sultamicillin, Talampicillin, Tazocillin, TicarcillinLoracarbef, 3-chloro-1-carbacephem, 3-thiocarbacephem, Flomoxef, Latamoxef, Latamoxef, Cefazolin, Cefalexin, Cefalotin, Cefradine, Ceftezole, Cefuroxime, Cefaclor, Cefamandole, Cefotiam, Cefonicid, Ceforanide, Cefoperazone, Ceftriaxone, Ceftazidime, Cefotaxime, Ceftizoxime, Cefixime, Cefodizime, Cefpiramide, Cefpirome, Cefepime, Cefclidin, Cefadroxil, Cefatrizine, Cefazedone, Cefcapene pivoxil, Cefolidin, Cefdinir, Cefditoren, Cefetamet, Cefmenoxime, Cefotetan, Cefozopran, Cefpimizole, Cefpodoxime Proxetil, Cefprozil, Cefroxadine, Cefsulodin, Cefteram, Ceftibuten, Ceftizoxime, Cefuzonam, Cephatril, Cefaloglycin, Cefaloridine, Cephalosporin, Cephalotin, Cephapirin, Cefbuperazone, Cefminox, Imipenem, Meropenem, Panipenem, Biapenem, Ertapenem, Faropenem, Cefoxitin, Cefmetazole, Aztreonam, Carumonam, Latamoxef, Flomoxef, clavulanic acid, clavulanate, clavulanate, Sulbactam, and Tazobactam.

The aminoglycoside antibiotics are preferably Streptomycin, Kanamycin, Gentamicin, Amikacin, Tobramycin, Netilmicin, Sisomicin, Albomycin, Arbekacin, Bambermycin, Butyrosin, Dibekacin, Dihydrostreptomycin, Fortimicin, Isepamicin, Micronomicin, Neomycin, Neodecyllin, Paromomycin, Ribostamycin, Spectinomycin, and Trospectomycin.

The macrolide antibiotics are preferably Erythromycin, Clarithromycin, Azithromycin, Roxithromycin, Carbomycin, Dirithromycin, Erythromycin Acistrate, Erythromycin Estolate, Erythromycin Gluceptate, Erythromycin Lactobionate, Erythromycin Stinoprate, Erythromycin stearate, Josamycin, Leucomycin, Midecamycin, Miokamycin, Oleandomycin, Primycin, Rokitamycin, Rosaramicin, Spiramycin, and Troleandomycin.

The lincomycin antibiotics are preferably Lincomycin and Clindamycin.

The polypeptide antibiotics are preferably Polymixin B, Polymixin E, Norvancomycin, Teicomycin, Vancomycin, Teicoplanin, Amphomycin, Bacitracin, Capreomycin, Colistin, Colomycin, Enduracidin, Enviomycin, Fusafungine, Gramicidin, Mikamycin, Polymixin, Pristinamycin, Dalfopristin, Ristocetin, Thiostrepton, Tuberactinomycin, Tyrocidine, Tyrothricina, Viomycin, Virginiamycin, and Zinc Bacitracin.

The tetracycline antibiotics are preferably Apicycline, Chlortetracycline, Clomocycline, Demeclocycline, Doxycycline, Guamecycline, Lymecycline, Meclocycline, Metacycline, Minocycline, Oxytetracycline, Penimepicycline, Pipacycline, Rolitetracycline, Sancycline, Tetracycline, Cycloserine, Mupirocin, and Tuberin.

The quinolone antibiotics are preferably Nalidixic Acid, Pipemidic Acid, Norfloxacin, Ofloxacin, Ciprofloxacin, Enoxacin, Pefloxacin, Levofloxacin, Gatifloxacin, Moxifloxacin, Norfloxacin, Fleroxacin, Lomefloxacin, Sparfloxacin, Grepafloxacin, Rufloxacin, Clinafloxacin, Balofloxacin, Trovafloxacin, Fluoroquinolone, Alatrofloxacin Mesylate, Cinoxacin, Difloxacin, Flumequine, Grepafloxacin, Miloxacin, Marbofloxacin, Nadifloxacin, Oxolinic acid, Pazufloxacin, Piromidic acid, Rosoxacin, Temafloxacin, Tosufloxacin, and Trovafloxacin mesilate.

The sulfanilamide are preferably Sulfamethoxazole, Trimethoprim, acetyl sulfamethoxypyridazine, Benzylsulfamide, Chloramine B, Chloramine T, Dichloramine T, Sulfisomidine, β-GlucosylSulfanilamide, Mafenide, 4'-methylaminosulfonyl-sulfonanilide, Noprylsulfamide, Phthalylsulfacetamide, Phthalylsulfathiazole, Salicylazosulfapyridine, Sulfasuxidine, Sulfabenzamide, Sulfacetamide, Sulfachlorpyridazine, Sulfachrysoidine, Sulfacitine, Sulfadiazine, Sulfadicramide, Sulfadimethoxine, Sulfadoxine, Sulfaethidole, Sulfaguanidine, Sulfaguanole, Sulfalene, Sulfaloxic Acid, Sulfamerazine, Oxymethylpyrimidine, Sulfamerazine, Sulfamethizole, Sulfametomidine, Sulfisoxazole, Sulfamethoxypyridazine, Sulfametrole, Sulfamidochrysoidine, Sulfanilamidozole, Sulfanilamide, 4-Sulfanilamidosalicylic acid, SulfanilamidoSulfanilamide, Sulfanilylurea, n-Sulfanilamido-3,4-carboxamide, Sulfanitran, Sulfaperin, Sulfaphenazole, Sulfaproxyline, Sulfapyrazole, Sulfapyridine, Sulfasomizole, Sulfasymazine, Sulfathiazole, Sulfathiourea, Sulfatolamide, Sulfisomidine, and Sulfatroxazole.

The furan antibiotics are preferably Nitrofurantoin, Furazolidone, Furaltadone, Furazolium Chloride, Nifuradene, Nifuratel, Nifurfoline, Nifurpirinol, Nifurprazine, and Nifurtoinol.

The nitroimidazole antibiotics are preferably Metronidazole, Tinidazole and Omidazole.

The anti-tuberculosis antibiotics are preferably Isoniazid, Rifampicin, Rifamide, Pyrazinamide and Ethambutol.

The antifungal antibiotics are preferably amphotericin B, fluconazole, Itraconazole, 5-Flucytosine, Candicidin, Dermostatin, Filipin, antifungal pigments, Hachimycin, Hamycin, Lucensomycin, Mepartricin, Pimaricin, Natamycin, Nysfungin, Pecilocin, Permycin, Azaserine, Griseofulvin, Oligomycin, Neodecyllin, Pyrrolnitrin, Siccanin, Tubercidin, Viridin, Allyl amine, Butenafine, Naftifine, Terbinafine, imidazole, Bifonazole, Butoconazole, Chlordantoin, Chlormidazole, Cloconazole, Clotrimazole, Econazole, Enilconazole, Fenticonazole, Flutrimazole, Isoconazole, Ketoconazole, Lanoconazole, Miconazole, Omoconazole, Oxiconazole nitrate, Sertaconazole, Sulconazole, Tioconazole, Voriconazole, thiocarbamate, Tolciclate, Tolindate, Tolnaftate, triazole, Saperconazole, Terconazole, Acrisorcin, Amorolfine, Xenysalate, Bromosalicylchloranilide, Buclosamide, Calcium propionate, Chlorphenesin, Coparaffinate, Dimazole dihydrochloride, Exalamide, Flucytosine, Haletazole, Hexetidine, lipopeptide such as Echinocandin, Loflucarban, Nifuratel, Potassium iodide, Propionic acid, 2-Mercaptopyridine oxide, Salicylanilide, Sodium propionate, Sulbentine, Tenonitrozole, Triacetin, Benzthiadiazine acetate, Undecenoic acid, and Zinc propionate.

The HAase has the activity of degrading hyaluronic acid under a neutral condition of pH 5.0-8.0.

HAase is an enzyme that degrades hyaluronic acid and reduces the viscosity of hyaluronic acid in extracellular matrix, thus increasing its tissue permeability. The HAase is selected from the group consisting of HAase extracted from animal testis, recombinant animal HAase or a mutant thereof, recombinant and/or extracted bacterial HAase, recombinant human HAase or a mutant thereof; preferably, the HAase is recombinant human HAase or a mutant thereof; further preferably, the HAase is recombinant human HAase or a mutant thereof; more preferably, the recombinant human HAase comprises an amino acid sequence as shown in SEQ ID NO. 1; and most preferably, the recombinant human HAase comprises an amino acid sequence as shown in SEQ ID NO. 2.

According to the HAase, recombinant human HAase or a mutant thereof useful in the present invention, the mutant is as described in CN1942588B, CN102943067B, CN102307993B, CN103205407B, CN104244968B, and CN111971387A respectively.

The activity of HAase can be defined by units/ml (U/mL) or by the total enzyme activity (U) in a specific preparation, as further explained below. The standard definition of one unit of enzyme activity (U) is the amount of enzyme that catalyzes the reaction of a defined amount of substrate per unit time, for example, one mole or one nanomole of substrate per minute. Techniques for determining the activity of HAase preparations are known in the art, and the activity of HAase preparations is usually expressed in U or units (expressed hereinafter in "units").

"HAase activity refers to the ability of HAase to catalytically cleave hyaluronic acid. The Chinese Pharmacopoeia provides a method for determining the HAase activity, indirectly by measuring the amount of remaining higher-molecular-weight hyaluronic acid substrate after the enzyme is allowed to catalytically cleave hyaluronic acid at 37°C for 30 min. A standard reference solution can be used in the assay to determine the relative activity (in units) of any HAase. In-vitro assays for the determination of HAase are known in the art. Exemplary assays include micro-turbidimetric assay described below, which indirectly measures the cleavage of hyaluronic acid by HAase by detecting an insoluble precipitate formed when uncleaved hyaluronic acid binds to serum albumin. The reference standard can be used, for example, to generate a standard curve, so as to determine the activity of the tested HAase in units.

The antibiotic pharmaceutical composition comprises one or more antibiotics and recombinant human HAase, as well as a pharmaceutically acceptable adjuvant.

The drug combination of the antibiotic and the recombinant human HAase can be combined into a compound preparation, or a kit consisting of separate packages of non-compound preparations.

The Cₘₐₓ of the antibiotic in the composition is increased by at least 10%, for example, at least 20%, at least 30%, at least 40%, and at least 50%, compared with that in the case where the antibiotic is administered alone; and/or the Tₘₐₓ of the antibiotic in the composition is reduced by at least 20%, for example, at least 30%, at least 40%, and at least 50%, compared with that in the case where the antibiotic is administered alone.

When the composition is subcutaneously administered, the reduction in the diversity of intestinal microbial flora relative to that before administration is 90% or less, for example, 80% or less, 70% or less, 60% or less, and 50% or less, of the reduction relative to the diversity before administration when the antibiotic is administered orally.

The composition further comprises a pharmaceutically acceptable adjuvant selected from the group consisting of a buffer, a stabilizer, a nonionic surfactant, a co-solvent, a preservative and/or an excipient. The buffer is selected from the group consisting of a histidine buffer, an acetic acid buffer, a phosphate buffer, a citric acid buffer and a Tris buffer. The stabilizer is selected from the group consisting of trehalose, sucrose, mannitol, sodium chloride, methionine and disodium edetate. The pH regulator is selected from the group consisting of phosphoric acid, acetic acid, hydrochloric acid, citric acid, sodium hydroxide and Tris. The nonionic surfactant is selected from the group consisting of polysorbate 20, polysorbate 80 and poloxamer 188. The co-solvent is selected from the group consisting of propylene glycol, ethanol, polyethylene glycol, glycerol, sodium dodecyl sulfate and cyclodextrin. The preservative is selected from the group consisting of glycerol, methyl paraben, propyl paraben, benzoic acid, sodium benzoate and ethanol. The excipient is selected from the group consisting of sorbitol, mannitol, trehalose, glycerol, lactose, sucrose, trehalose, maltose and glucose.

The concentration of the buffer is 1-100 mM, such as 5 mM, preferably 10 mM or 50 mM; and/or
the concentration of the nonionic surfactant is 0.01-0.5% (w/v), preferably 0.02% (w/v), and/or
the concentration of the co-solvent is 0.01 g/L-100 g/L; and/or
the concentration of the preservative is 0.1 g/L-200 g/L; and/or
the concentration of the excipient is 1 g/L-200 g/L.

The drug combination can be a lyophilized preparation and/or a liquid preparation. Preferably, the combination of the antibiotic and the recombinant human HAase can be combined into a compound preparation or non-compound separate preparations.

### 3. Kit and preparation method of the kit

In a second aspect, the present invention provides a kit, comprising the pharmaceutical composition as described above, where the antibiotic and the HAase in the kit are packaged in mixture or separately.

The separately packaged antibiotic and/or the separately packaged HAase are lyophilized preparations or liquid preparations. The lyophilized preparation is reconstituted before administration to a subject, and the liquid preparation is directly administered to the subject or diluted before administration to the subject.

The antibiotic and the HAase are administered sequentially or simultaneously, and the separately packaged antibiotic and/or the separately packaged HAase are administered sequentially or simultaneously.

The antibiotic and the HAase are administered sequentially through a three-way infusion tube, and the administration speed is preferably controlled by manually pushing, by an infusion pump, or by gravity.

In a first embodiment, the kit includes two single-dose containers. A first container contains a single particular dose of the liquid preparation comprising 45 units/ml to -4500000 units/ml recombinant human HAase, and a second container contains a fixed single dose of the therapeutic agent.

The container containing the liquid preparation of recombinant human HAase is selected from a vial and a prefilled syringe, and preferably, a prefilled syringe.

Preferably, the liquid preparation of recombinant human HAase includes 45 units/ml to 4500000 units/ml recombinant human HAase.

The volume of the liquid preparation of recombinant human HAase may be, 0.1 -50 ml, for example, 0.2 ml, 0.5 ml, 1 ml, 1.5 ml, 2 ml, 2.50 ml, 5.00 ml, 10.00 ml, 15.00 ml, 20 ml, 30 ml, 40 ml or 50 ml.

In some embodiments, 1 ml of the liquid preparation of recombinant human HAase includes 45 units to 75000 units of recombinant human HAase.

The first container is a vial, the second container is a vial, and the third container is a vial or a prefilled syringe.

The first container is a vial or a prefilled syringe, and preferably a prefilled syringe; and the second container is a vial.

The liquid preparation of recombinant human HAase preferably comprises 45 unit/ml to -100000 unit/ml recombinant human HAase; and further preferably 45 unit/ml to -50000 unit/ml recombinant human HAase.

A third aspect of the present invention provides a method for preparing the kit as described above, which includes:
(a) providing an antibiotic;
(b) providing HAase; and
(c) optionally providing a pharmaceutically acceptable adjuvant.

The antibiotic and the HAase are respectively prepared into a lyophilized preparation or a liquid preparation; or the antibiotic and the HAase are mixed and then prepared into a lyophilized preparation or a liquid preparation.

In a fourth aspect, the present invention relates to a method for treating a disease by administering the kit according to the second aspect, by administrating separately or in mixture.

The separate administration includes the following steps:
(a) intradermally or subcutaneously administrating the liquid preparation of recombinant human HAase in the kit to the subject; and
(b) administrating the therapeutic agent in the kit to the subject.

Steps (a) and (b) can be performed separately, simultaneously or alternatively.

When Steps (a) and (b) are performed separately, the time interval between Steps (a) and (b) is 0 to 24 hrs, and
preferably, the time interval is 0 min, at most 1 min, at most 2 min, at most 3 min, at most 4 min, at most 5 min, at most 6 min, at most 7 min, at most 8 min, at most 9 min, at most 10 min, at most 15 min, at most 20 min, at most 25 min, at most 30 min, at most 1 hr, at most 2 hrs, at most 3 hrs, at most 6 hrs, at most 12 hrs or at most 24 hrs.

In the case of sequential subcutaneous administration, the time interval between Steps (a) and (b) may be 0 min, 1 min, 2 min, 3 min, 4 min, 5 min, 6 min, 7 min, 8 min, 9 min, 10 min, 15 min, 20 min, 25 min, 30 min, 35 min, 40 min, 45 min, 50 min, 55 min or 60 min.

In the case of separate administration, simultaneous administration or sequential administration can be realized by a three-way valve.

Upon separate administration, the administration rate is controlled by an injection pump or by gravity,

The liquid preparation of recombinant human HAase can be dosed at a rate of 0.1 to 2 ml/min.

The therapeutic agent can be injected at a rate of 5 ml/hr, 10 ml/hr, 30 ml/hr, 60 ml/hr, 120 ml/hr, 240 ml/hr or 300 ml/hr.

The administration in mixture comprises mixing the liquid preparation of recombinant human HAase and the therapeutic agent in the kit and subcutaneously administrating the mixture to the subject. 1 mL of the liquid preparation comprises 45 units-500000 units recombinant human HAase.

Upon administration in mixture, the administration rate is controlled by an injection pump or by gravity,

After the liquid preparation of recombinant human HAase and the therapeutic agent are mixed, the mixture can be injected at a rate of 5-300 ml/hr, for example, 5 ml/hr, 10 ml/hr, 30 ml/hr, 60 ml/hr, 120 ml/hr, 240 ml/hr or 300 ml/hr.

The therapeutic agent intended to be mixed is in the form of a liquid or a dry powder.

The liquid preparation of recombinant human HAase can be administered directly and/or administered after dilution.

The diluent can be conventional in the art, for example, physiological saline.

The dilution factor can be conventional in the art, for example, 1:10.

### 4. Use in the preparation of drugs and injection system

The present invention further relates to use of the composition and kit comprising an antibiotic and HAase in the preparation of drugs for treating diseases. In a specific embodiment, the diseases are selected from the group consisting of infections with bacteria, fungi, actinomycetes, mycoplasma, chlamydia, spirochete, and amoeba and diseases caused by the infections.

Preferably, the diseases are diseases caused by bacterial or fungal infection. The bacteria include Escherichia, Pseudomonas, Klebsiella, Acinetobacter, Enterobacter, Citrobacter, Haemophilus, Proteus, Salmonella, Shigella, Serratia, Corynebacterium, Staphylococcus, Streptococcus, Enterococcus, Neisseria, Mycobacterium, and Legionella. The fungi include Trichophyton, Epidermophyton, Microsporum, Candida, Cryptococcus, Coccidioides, Histoplasma, Sporothrix, Blastomyces, Geotrichum, Aspergillus, Mucor and Penicillium.

Preferably, the diseases are diseases caused by bacterial or fungal infection, and the bacteria or fungi are Haemophilus influenzae type B, Pseudomonas aeruginosa types A and B, Staphylococcus aureus, Streptococcus group B, Streptococcus pneumoniae types (1, 3, 4, 6, 7, 8, 9, 12, 14, 18, 19 and 23), Staphylococcus epidermidis, Staphylococcus saprophyticus, Streptococcus pyogenes, Streptococcus viridans, Streptococcus agalactiae, Diplococcus pneumoniae, Bacillus anthracis, Corynebacterium diphtheriae, Bordetella pertussis, Bacillus tetani, Mycobacterium tuberculosis, Bacillus, Clostridium perfringens, Mycobacterium leprae, Gonococcus, Meningococcus, Legionella pneumophila, Shigella dysenteriae, Pseudomonas aeruginosa, Bacillus proteus, Vibrio cholerae, Vibrio parahaemolyticus, Escherichia coli, Salmonella typhi, Salmonella paratyphi, Salmonella, Haemophilus influenzae, Acinetobacter, Aspergillus, Candida, Cryptococcus, Mucor or Fusarium.

The disease caused by the infection with pathogenic microorganisms includes chronic osteomyelitis caused by Staphylococcus aureus, endocarditis caused by drug-resistant Staphylococcus aureus or Enterococcus, typhoid fever, paratyphoid fever, bacterial food poisoning, bacterial infectious diarrhea, cholera, bacillary dysentery, brucellosis, plague, anthrax, diphtheria, pertussis, scarlatina, epidemic cerebrospinal meningitis, tuberculosis, bacterial blood infection, bacterial upper respiratory tract infection, bacterial lower respiratory tract infection, bacterial urinary tract infection, bacterial abdominal cavity infection, dermatophytosis, mycotic stomatitis, candida vaginitis, fungal pneumonia, fungal urinary tract infection, fungemia, cryptococcosis, candidiasis, aspergillosis, and pneumocystis.

Use of the pharmaceutical composition comprising the preparations above in the preparation of drugs for treating diseases is provided.

The present invention further relates to a container and an injection system. The packaging material includes, but is not limited to, a vial, a syringe or a test tube. The injection system includes, but is not limited to, a syringe, an injection pump, an injection pen, a needle-free device or a subcutaneous patch delivery device.

The components in the injection device may be conventional, and includes a container, a sealing element and an injection needle.

The container includes, but is not limited to, a vial, a syringe or a test tube.

The material of the container may be conventional in the art, for example, glass or plastic.

The sealing element includes, but is not limited to, a sealing plug or a sealing ring.

The material of the sealing element may be conventional in the art, for example, rubber, plastic or a polymer material.

The injection needle includes, but is not limited to, a liquid injection needle, a single needle and a microneedle array.

The material of the injection needle may be conventional in the art, for example, metal, silicon, silicon dioxide, glass, nickel, titanium or a biodegradable polymer.

The liquid injection includes, but is not limited to, a liquid injection containing penicillin bottle, a liquid injection containing ampoule, or a prefilled injection system.

The ampoule for liquid injection may be a glass ampoule or a plastic ampoule.

The prefilled injection system can be conventional, for example, a prefilled syringe in the art.

Preferably, the container is a penicillin bottle made of neutral borosilicate glass with a specification of 0.1 to 20 ml.

The sealing element is a sealing plug made of halogenated butyl rubber.

The injection needle is a single needle or a microneedle array.

Preferably, the material of the single needle can be 304 or 316 stainless steel, with a specification of 30G, 24G, 27G or 29G. The microneedle array can be made of 304 or 316 stainless steel, a biodegradable polymer, and includes nano-sized needles with a height of 10 to 2000 µm and a width of 10 to 50 µm.

In one embodiment of the present invention, a preparation is provided, which comprises a pharmaceutical composition according to the first aspect of the present invention and instructions for use. The preparation further includes a container. The preparation may further include other materials desired from the commercial and user's point of view, including other buffers, diluents, filters, needles, syringes, infusion pumps and packaging inserts printed with instructions for use.

On the basis of common knowledge in the art, the above-mentioned preferred conditions can be combined at will, to obtain various preferred examples of the present invention.

The reagents and raw materials used in the present invention are all commercially available.

The present invention has the following positive and progressive effects. In the present invention, the problem of subcutaneous administration of antibiotics is solved. The bioavailability of antibiotics can be up to 50% or higher. The clinical use is not limited by volume, and the administration is much convenient, which improve the treatment experience of patients and reduce the side effects related to infusion. The patient has high comfort, and the drug acts quickly, has high blood concentration, and will not cause gastrointestinal microbial disturbance.

### Examples

### Example 1: Preparation of recombinant human HAase

CHO cells stably expressing the recombinant human HAase protein were cultured in suspension in an independently researched and developed serum-free medium, then subjected to fed-batch culture controlled by fluidically adding the independently researched and developed serum-free feed medium and then to shake-flask culture to gradually expand to a 30L-reactor scale.

After 3 to 4 days of culture, the amount of feed medium added to the bioreactor every day was 2% to 5% of the actual culture volume in the bioreactor. The culture temperature was controlled at 35°C to 37°C, and the pH was controlled at 7.0 by adding 10% Na₂CO₃ and CO₂. The aeration rate of the reactor was controlled at 0.015 to 0.15 vvm, the rotational speed was controlled at 80 to 150 rpm; and the dissolved oxygen was controlled at 20% to 40%. During cell culture, a sample was taken every day to monitor the temperature, pH, glucose concentration, lactic acid concentration, osmolarity and protein expression. When the viability of CHO cells was lower than 80% or the culture period reached 14 to 20 days, the culture was ended and a supernatant containing recombinant human HAase was obtained.

The obtained supernatant containing recombinant human HAase was sequentially subjected to depth filtration, anion chromatography, affinity chromatography, hydrophobic chromatography, cation chromatography, and ultrafiltration. Following this process, three batches of stock solutions were produced, and a recombinant human HAase stock solution with an SEC purity of over 95%, and HAase with an RP purity of over 85% were obtained, having an enzyme activity of 70000 units/mg or higher.

### Example 2. Compatibility study of HAase and Meropenem in solution

The compatibility of HAase and an antibiotic was studied by preparing a solution comprising the two substances. A dry Meropenem power for injection was dissolved in physiological saline to give a concentration of 5 mg/ml and 50 mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Meropenem does not interfere with the activity of HAase. Thus, studies on the mixed preparation of HAase and Meropenem can be further carried out.

**Table 1. Compatibility study of Meropenem and HAase**

| Experimental group | Final concentration of HAase (units/ml) | | | |
|---|---|---|---|---|
| | 150 | 500 | 2000 | 6000 |
| 5 mg/ml Meropenem | 150 | 511 | 1979 | 6271 |
| 50 mg/ml Meropenem | 145 | 491 | 1984 | 6121 |
| Physiological saline | 161 | 488 | 1941 | 6020 |

### Example 3. Compatibility study of HAase and Ceftriaxone in solution

The compatibility of HAase and an antibiotic was studied by preparing a solution comprising the two substances. A dry Ceftriaxone sodium power for injection was dissolved in physiological saline to give a concentration of 50 mg/ml and 250 mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Ceftriaxone does not interfere with the activity of HAase. Thus, studies on the mixed preparation of HAase and Ceftriaxone can be further carried out.

**Table 2. Compatibility study of Ceftriaxone and HAase**

| Experimental group | Final concentration of HAase (units/ml) | | | |
|---|---|---|---|---|
| | 150 | 500 | 2000 | 6000 |
| 50 mg/ml Ceftriaxone | 162 | 510 | 1986 | 6067 |
| 250 mg/ml Ceftriaxone | 162 | 507 | 1962 | 6232 |
| Physiological saline | 161 | 488 | 1941 | 6020 |

### Example 4. Compatibility study of HAase and Azithromycin in solution

The compatibility of HAase and an antibiotic was studied by preparing a solution comprising the two substances. A dry Azithromycin power for injection was dissolved in physiological saline to give a concentration of 10 mg/ml and 100 mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Azithromycin does not interfere with the activity of HAase. Thus, studies on the mixed preparation of HAase and Azithromycin can be further carried out.

**Table 3. Compatibility study of Azithromycin and HAase**

| Experimental group | Final concentration of HAase (units/ml) | | | |
|---|---|---|---|---|
| | 150 | 500 | 2000 | 6000 |
| 10 mg/ml Azithromycin | 152 | 482 | 1651 | 6140 |
| 100 mg/ml Azithromycin | 140 | 500 | 1760 | 5818 |
| Physiological saline | 161 | 488 | 1941 | 6020 |

### Example 5. Compatibility study of HAase and Cefradine in solution

The compatibility of HAase and an antibiotic was studied by preparing a solution comprising the two substances. A dry Cefradine powder for injection was dissolved in physiological saline to give a concentration of 50 mg/ml and 250 mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Cefradine does not interfere with the activity of HAase. Thus, studies on the mixed preparation of HAase and Cefradine can be further carried out.

**Table 4. Compatibility study of Cefradine and HAase**

| Experimental group | Final concentration of HAase (units/ml) | | | |
|---|---|---|---|---|
| | 150 | 500 | 2000 | 6000 |
| 50 mg/ml Cefradine | 162 | 492 | 2009 | 6126 |
| 250 mg/ml Cefradine | 161 | 482 | 2080 | 5797 |
| Physiological saline | 161 | 488 | 1941 | 6020 |

### Example 6. Compatibility study of HAase and Teicoplanin in solution

The compatibility of HAase and an antibiotic was studied by preparing a solution comprising the two substances. A dry Teicoplanin powder for injection was dissolved in physiological saline to give a concentration of 10 mg/ml and 70 mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Teicoplanin does not interfere with the activity of HAase. Thus, studies on the mixed preparation of HAase and Teicoplanin can be further carried out.

**Table 5. Compatibility study of Teicoplanin and HAase**

| Experimental group | Final concentration of HAase (units/ml) | | | |
|---|---|---|---|---|
| | 150 | 500 | 2000 | 6000 |
| 10 mg/ml Teicoplanin | 160 | 488 | 1972 | 6033 |
| 70 mg/ml Teicoplanin | 152 | 498 | 1961 | 5979 |
| Physiological saline | 161 | 488 | 1941 | 6020 |

### Example 7. Compatibility study of HAase and Ceftazidime in solution

The compatibility of HAase and an antibiotic was studied by preparing a solution comprising the two substances. A dry Ceftazidime powder for injection was dissolved in physiological saline to give a concentration of 20 mg/ml and 200 mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Ceftazidime does not interfere with the activity of HAase. Thus, studies on the mixed preparation of HAase and Ceftazidime can be further carried out.

**Table 6. Compatibility study of Ceftazidime and HAase**

| Experimental group | Final concentration of HAase (units/ml) | | | |
|---|---|---|---|---|
| | 150 | 500 | 2000 | 6000 |
| 20 mg/ml Ceftazidime | 145 | 501 | 2026 | 6027 |
| 200 mg/ml Ceftazidime | 148 | 488 | 2001 | 5981 |
| Physiological saline | 161 | 488 | 1941 | 6020 |

### Example 8. Compatibility study of HAase and Ertapenem in solution

The compatibility of HAase and an antibiotic was studied by preparing a solution comprising the two substances. A dry Ertapenem power for injection was dissolved in physiological saline to give a concentration of 30mg/ml and 300mg/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Ertapenem not interfere with the activity of HAase. Thus, studies on the mixed preparation of HAase and Ertapenem can be further carried out.

**Table 7. Compatibility study of Ertapenem and HAase**

| Experimental group | Final concentration of HAase (units/ml) | | | |
|---|---|---|---|---|
| | 150 | 500 | 2000 | 6000 |
| 30 mg/ml Ertapenem | 152 | 501 | 2016 | 6121 |
| 300 mg/ml Ertapenem | 156 | 511 | 1974 | 5990 |
| Physiological saline | 161 | 488 | 1941 | 6020 |

### Example 9. Compatibility study of HAase and Polymyxin B in solution

The compatibility of HAase and an antibiotic was studied by preparing a solution comprising the two substances. A dry Polymyxin B powder for injection was dissolved in physiological saline to give a concentration of 50,000 units/ml and 250,000 units/ml, and then HAase was added to give a final concentration of 150, 500, 2000, and 6000 units/ml respectively. After preparation, the solution was left to stand at room temperature for 15 min. The appearance of the solution was observed, and the concentration of HAase was determined.

The results show that after the 8 solutions are prepared, the solutions are clear and transparent, and no precipitation is found. The activity of HAase in the solution was determined. The results show that Polymyxin B does not interfere with the activity of HAase. Thus, studies on the mixed preparation of HAase and Polymyxin B can be further carried out.

**Table 8. Compatibility study of Polymyxin B and HAase**

| Experimental group | Final concentration of HAase (units/ml) | | | |
|---|---|---|---|---|
| | 150 | 500 | 2000 | 6000 |
| 50,000 units/ml Polymyxin B | 149 | 488 | 2126 | 5899 |
| 250,000 units/ml Polymyxin B | 151 | 501 | 2014 | 6090 |
| Physiological saline | 161 | 488 | 1941 | 6020 |

### Example 10: Study of liquid preparations of HAase

The medium in the recombinant human HAse stock solution obtained in Example 1 was replaced by liquid preparations of different compositions, and the recombinant human HAase was adjusted to a desired concentration. All preparations were sterilized and filtered through a 0.22 µm low protein adsorption filter, and filled into a sterilized 5 ml glass vial under aseptic conditions. The vial was plugged with a fluororesin coated butyl rubber stopper and capped with an aluminum/plastic flip-off seal. The filling volume was 2 ml. These preparations were stored at different temperatures, and samples were taken at specified time intervals to investigate the stability of the preparations. The stability data of different preparations were summarized. Samples were subjected to accelerated test at 25°C, and sampled at different times to analyze the HAase for SEC purity, RP purity and enzyme activity. The test results are shown in Tables 2-6.

As shown in Table 9, after standing for a period of time at 25°C, the RP purity and enzyme activity of the recombinant human HAase in the presence of 2 mM methionine decrease obviously; and the preparations of recombinant human HAase with 5, 10 and 50 mM methionine have good stability.

As shown in Table 10, after standing for a period of time at 25°C, with a stabilizer that comprises 250 mM trehalose and 250 mM sucrose, the SEC purity and enzyme activity of the recombinant human HAase decrease obviously; and with a stabilizer that comprises 25, 53 or 200 mM trehalose, and 25, 53 or 200 mM sucrose, the preparations of recombinant human HAase have good stability.

As shown in Table 11, with a surfactant that is polysorbate 20 having a concentration of 0.01 to 0.1%, polysorbate 80 having a concentration of 0.01 to 0.1%, or Poloxamer 188 having a concentration of 0.01 to 0.1%, the preparations of recombinant human HAase have good stability.

As shown in Table 12, with an excipient that comprises 160 to 280 mM mannitol, the preparations of recombinant human HAase have good stability.

As shown in FIG. 13, the recombinant human HAase activity in the liquid preparation of recombinant human HAase in this example is stable at 150, 500, 1, 000, 5, 000, 50, 000, 300, 000, 1, 500, 000, 3, 000, 000 and 4, 500, 000 units/ml, and the enzyme activity can still be maintained at 95% or higher after storage for 12 months at 2 to 8°C.

**Table 9**

| | | | 1 | 2 | 3 | 4 |
|---|---|---|---|---|---|---|
| Ingredients in liquid preparation | Recombinant human HAase | | 5000 units/ml | 5000 units/ml | 5000 units/ml | 5000 units/ml |
| | Phosphate buffer. | | 10 mM | 10 mM | 10 mM | 10 mM |
| | Trehalose | | 53 mM | 53 mM | 53 mM | 53 mM |
| | Sodium chloride | | 130 mM | 130 mM | 130 mM | 130 mM |
| | Methionine | | 5 mM | 10 mM | 50 mM | 2 mM |
| | Polysorbate 20 | | 0.02% | 0.02% | 0.02% | 0.02% |
| | pH | | 7.0 | 7.0 | 7.0 | 7.0 |
| Stability data | Initial | SEC purity | 99.5% | 99.7% | 99.8% | 99.6% |
| | | RP purity | 94.8% | 94.6% | 95.3% | 94.2% |
| | | Enzyme activity | 5124 units/ml | 5035 units/ml | 5118 units/ml | 5083 units/ml |
| | 1 month at 25°C | SEC purity | 99.2% | 99.4% | 99.4% | 99.2% |
| | | RP purity | 94.4% | 93.9% | 95.0% | 93.5% |
| | | Enzyme activity | 5202 units/ml | 5147 units/ml | 4998 units/ml | 4943 units/ml |
| | 3 month at 25°C | SEC purity | 99.3% | 99.0% | 99.2% | 99.0% |
| | | RP purity | 93.8% | 94.0% | 94.5% | 90.9% |
| | | Enzyme activity | 5017 units/ml | 5113 units/ml | 5012 units/ml | 4781 units/ml |

**Table 10**

| | | | 1 | 2 | 3 | 4 | 5 | 6 | 7 | 8 |
|---|---|---|---|---|---|---|---|---|---|---|
| Ingredients in liquid preparation | Recombinant human HAase | | 5000 units/ml | 5000 units/ml | 5000 units/ml | 5000 units/ml | 5000 units/ml | 5000 units/ml | 5000 units/ml | 5000 units/ml |
| | Phosphate buffer. | | 10mM | 10mM | 10mM | 10mM | 10mM | 10mM | 10mM | 10mM |
| | Trehalose | | 200mM | 53mM | 25mM | / | / | / | 250mM | / |
| | Sucrose | | / | / | / | 200mM | 53mM | 25mM | / | 250mM |
| | Sodium chloride | | 30mM | 130mM | 180mM | 30mM | 130mM | 180mM | 20mM | 20mM |
| | Methionine | | 10mM | 10mM | 10mM | 10mM | 10mM | 10mM | 10mM | 10mM |
| | Polysorbate 20 | | 0.02% | 0.02% | 0.02% | 0.02% | 0.02% | 0.02% | 0.02% | 0.02% |
| | pH | | 7.0 | 7.0 | 7.0 | 7.0 | 7.0 | 7.0 | 7.0 | 7.0 |
| Stability data | Initial | SEC purity | 99.4% | 99.7% | 99.6% | 99.8% | 99.5% | 99.4% | 99.7% | 99.5% |
| | | RP purity | 94.8% | 95.5% | 95.1% | 94.7% | 95.5% | 94.6% | 94.7% | 95.7% |
| | | Enzyme activity | 5193 units/ml | 5042 units/ml | 5183 units/ml | 5029 units/ml | 5016 units/ml | 5149 units/ml | 5034 units/ml | 5017 units/ml |
| | 1 month at 25°C | SEC purity | 99.3% | 99.7% | 99.4% | 99.7% | 99.3% | 99.2% | 98.9% | 98.5% |
| | | RP purity | 94.7% | 95.2% | 94.6% | 94.8% | 95.2% | 94.4% | 93.7% | 93.9% |
| | | Enzyme activity | 5072 units/ml | 5144 units/ml | 5091 units/ml | 5117 units/ml | 5046 units/ml | 5192 units/ml | 4761 units/ml | 4845 units/ml |
| | 3 month at 25°C | SEC purity | 99.1% | 99.4% | 99.2% | 99.6% | 99.4% | 99.4% | 97.4% | 97.1% |
| | | RP purity | 94.8% | 95.3% | 94.3% | 93.9% | 94.2% | 93.8% | 93.5% | 93.6% |
| | | Enzyme activity | 5228 units/ml | 5096 units/ml | 5203 units/ml | 5193 units/ml | 5063 units/ml | 5189 units/ml | 4592 units/ml | 4355 units/ml |

**Table 11**

| | | | 1 | 2 | 3 | 4 | 5 | 6 | 7 | 8 | 9 |
|---|---|---|---|---|---|---|---|---|---|---|---|
| Ingredients in liquid preparation | Recombinant human HAase | | 5000 units/ml | 5000 units/ml | 5000 units/ml | 5000 units/ml | 5000 units/ml | 5000 units/ml | 5000 units/ml | 5000 units/ml | 5000 units/ml |
| | Phosphate buffer | | 10mM | 10mM | 10mM | 10mM | 10mM | 10mM | 10mM | 10mM | 10mM |
| | Trehalose | | 53mM | 53mM | 53mM | 53mM | 53mM | 53mM | 53mM | 53mM | 53mM |
| | Sodium chloride | | 130mM | 130mM | 130mM | 130mM | 130mM | 130mM | 130mM | 130mM | 130mM |
| | Methionine | | 10mM | 10mM | 10mM | 10mM | 10mM | 10mM | 10mM | 10mM | 10mM |
| | Polysorbate 20 | | 0.01% | 0.02% | 0.10% | / | / | / | / | / | / |
| | Polysorbate 80 | | / | / | / | 0.01% | 0.02% | 0.1% | / | / | / |
| | Poloxamer 188 | | / | / | / | / | / | / | 0.01% | 0.02% | 0.1% |
| | pH | | 7.0 | 7.0 | 7.0 | 7.0 | 7.0 | 7.0 | 7.0 | 7.0 | 7.0 |
| Stability data | Initial | SEC purity | 99.3% | 99.5% | 99.7% | 99.2% | 99.7% | 99.4% | 99.4% | 99.6% | 99.6% |
| | | RP purity | 95.1% | 94.7% | 95.5% | 94.6% | 94.7% | 95.7% | 95.5% | 94.2% | 95.3% |
| | | Enzyme activity | 5203 units/ml | 5189 units/ml | 5233 units/ml | 5071 units/ml | 5117 units/ml | 5186 units/ml | 5192 units/ml | 5075 units/ml | 5034 units/ml |
| | 1 month at 25°C | SEC purity | 99.1% | 99.2% | 99.4% | 98.6% | 99.2% | 98.9% | 99.2% | 99.4% | 99.4% |
| | | RP purity | 94.8% | 94.5% | 94.6% | 94.6% | 94.2% | 95.2% | 95.0% | 94.4% | 95.1% |
| | | Enzyme activity | 5089 units/ml | 5037 units/ml | 5158 units/ml | 5263 units/ml | 5177 units/ml | 5195 units/ml | 5034 units/ml | 5203 units/ml | 5069 units/ml |
| | 3 month at 25°C | SEC purity | 98.9% | 99.0% | 99.0% | 98.7% | 99.0% | 98.8% | 99.3% | 98.6% | 98.6 % |
| | | RP purity | 94.4% | 94.7% | 93.8% | 93.6% | 94.0% | 94.8% | 94.7% | 94.0% | 94.3% |
| | | Enzyme activity | 5211 units/ml | 5010 units/ml | 5084 units/ml | 5174 units/ml | 5193 units/ml | 5244 units/ml | 5072 units/ml | 5181 units/ml | 5003 units/ml |

**Table 12**

| | | | 1 | 2 | 3 | 4 | 5 | 6 | 7 | 8 | 9 |
|---|---|---|---|---|---|---|---|---|---|---|---|
| Ingredients in liquid preparation | | Recombinant human HAase | 5000 units/ml | 5000 units/ml | 5000 units/ml | 5000 units/ml | 5000 units/ml | 5000 units/ml | 5000 units/ml | 5000 units/ml | 5000 units/ml |
| | | Phosphate buffer | 10mM | 10mM | 10mM | 10mM | 10mM | 10mM | 10mM | 10mM | 10mM |
| | | Trehalose | 53mM | 53mM | 53mM | 25mM | 53mM | 100mM | / | / | / |
| | Sucrose | | / | / | / | / | / | / | 25mM | 53mM | 100mM |
| | Mannitol | | 160mM | 220mM | 280mM | 220mM | 220mM | 220mM | 220mM | 220mM | 220mM |
| | Methionine | | 10mM | 10mM | 10mM | 10mM | 10mM | 10mM | 10mM | 10mM | 10mM |
| | Polysorbate 20 | | 0.02% | 0.02% | 0.02% | 0.02% | 0.02% | 0.02% | 0.02% | 0.02% | 0.02% |
| | pH | | 7.0 | 7.0 | 7.0 | 7.0 | 7.0 | 7.0 | 7.0 | 7.0 | 7.0 |
| Stability data | Initial | SEC purity | 99.3% | 99.1% | 99.4% | 99.1% | 99.7% | 99.5% | 99.3% | 99.3% | 99.6% |
| | | RP purity | 95.2% | 95.1% | 95.5% | 95.2% | 94.9% | 94.5% | 95.1% | 95.2% | 95.3% |
| | | Enzyme activity | 5011 units/ml | 5182 units/ml | 5077 units/ml | 5026 units/ml | 5122 units/ml | 5109 units/ml | 5072 units/ml | 5162 units/ml | 5099 units/ml |
| | 1 month at 25°C | SEC purity | 99.1% | 99.2% | 99.3% | 99.1% | 99.4% | 99.3% | 99.2% | 99.2% | 99.1% |
| | | RP purity | 95.5% | 94.7% | 94.9% | 94.2% | 94.6% | 95.1% | 94.5% | 94.9% | 95.2% |
| | | Enzyme activity | 5122 units/ml | 5023 units/ml | 5063 units/ml | 5017 units/ml | 5178 units/ml | 5023 units/ml | 5057 units/ml | 5088 units/ml | 5122 units/ml |
| | 3 months at 25°C | SEC purity | 98.8% | 98.9% | 98.4% | 98.2% | 98.8% | 98.8% | 99.1% | 99.0% | 98.3% |
| | | RP purity | 94.1% | 93.6% | 93.4% | 94.1% | 93.6% | 94.3% | 94.1% | 94.2% | 94.9% |
| | | Enzyme activity | 5076 units/ml | 5011 units/ml | 5052 units/ml | 5015 units/ml | 5098 units/ml | 5026 units/ml | 5082 units/ml | 5055 units/ml | 5021 units/ml |

**Table 13**

| | | | 1 | 2 | 3 | 4 | 5 | 6 | 7 | 8 | 9 |
|---|---|---|---|---|---|---|---|---|---|---|---|
| Ingredients in liquid preparation | Recombinant human HAase | | 150 units/ml | 500 units/ml | 1000 units/ml | 5000 units/ml | 50000 units/ml | 300000 units/ml | 1500000 units/ml | 3000000 units/ml | 4500000 units/ml |
| | Phosphate buffer | | 10mM | 10mM | 10mM | 10mM | 10mM | 10mM | 10mM | 10mM | 10mM |
| | Trehalose | | 53mM | 53mM | 53mM | 53mM | 53mM | 53mM | 53mM | 53mM | 53mM |
| | Sodium chloride | | 130mM | 130mM | 130mM | 130mM | 130mM | 130mM | 130mM | 130mM | 130mM |
| | Methionine | | 10mM | 10mM | 10mM | 10mM | 10mM | 10mM | 10mM | 10mM | 10mM |
| | Polysorbate 20 | | 0.02% | 0.02% | 0.02% | 0.02% | 0.02% | 0.02% | 0.02% | 0.02% | 0.02% |
| | pH | | 7.0 | 7.0 | 7.0 | 7.0 | 7.0 | 7.0 | 7.0 | 7.0 | 7.0 |
| Stability data | Initial | SEC purity | 99.3% | 99.4% | 99.5% | 99.2% | 99.3% | 99.1% | 99.4% | 99.2% | 99.1% |
| | | RP purity | 94.7% | 94.8% | 95.5% | 94.7% | 94.3% | 94.2% | 94.5% | 94.2% | 95.3% |
| | | Enzyme activity | 158 units/ml | 519 units/ml | 1184 units/ml | 5254 units/ml | 51869 units/ml | 302448 units/ml | 1548963 units/ml | 3085494 units/ml | 4564219 units/ml |
| | 1 month at 25°C | SEC purity | 99.1% | 99.2% | 99.2% | 99.0% | 99.2% | 99.2% | 98.8% | 99.1% | 98.7% |
| | | RP purity | 94.5% | 94.7% | 95.2% | 94.7% | 94.3% | 94.2% | 94.5% | 94.2% | 95.3% |
| | | Enzyme activity | 162 units/ml | 508 units/ml | 1042 units/ml | 5208 units/ml | 52076 units/ml | 308326 units/ml | 1523882 units/ml | 3040227 units/ml | 4578470 units/ml |
| | 3 months at 25°C | SEC purity | 98.8% | 99.1% | 99.3% | 98.6% | 98.9% | 99.0% | 98.9% | 98.8% | 99.0% |
| | | RP purity | 94.1% | 94.4% | 95.1% | 94.3% | 94.4% | 94.1% | 94.0% | 94.3% | 95.0% |
| | | Enzyme activity | 154 units/ml | 509 units/ml | 1119 units/ml | 5187 units/ml | 50438 units/ml | 304920 units/ml | 1537042 units/ml | 3068021 units/ml | 4527784 units/ml |
| | 12 months at 2-8°C | SEC purity | 98.9% | 99.0% | 99.2% | 98.8% | 98.7% | 99.1% | 98.8% | 98.7% | 98.9 % |
| | | RP purity | 94.3% | 94.2% | 95.0% | 94.2% | 94.0% | 94.1% | 94.2% | 94.1% | 94.8% |
| | | Enzyme activity | 155 units/ml | 511 units/ml | 1135 units/ml | 5194 units/ml | 50178 units/ml | 303901 units/ml | 1518413 units/ml | 3062403 units/ml | 4510369 units/ml |

### Example 11: Study of lyophilized powder preparations of HAase

According to the compositions of the preparations in Example 10, 7 representative HAase preparations were screened out, as shown in Table 14. 4 concentrations of HAase were designed for each composition, that is, 150, 500, 2000, and 6000 units/ml respectively. After lyophilization according to the lyophilization procedure shown in Table 15, the lyophilized powder was mixed with Meropenem.

### Example 14: Composition of lyophilized powder preparations of HAase

| | A | B | C | D | E | F | G |
|---|---|---|---|---|---|---|---|
| Disodium hydrogen phosphate | 10mM | 10mM | 10mM | 10mM | 10mM | 10mM | 10mM |
| Methionine | 10mM | 10mM | 10mM | 10mM | 10mM | 10mM | 10mM |
| Tween20 | 0.02% | 0.02% | 0.02% | 0.02% | 0.02% | 0.02% | 0.02% |
| Sodium chloride | 30mM | 130mM | -- | -- | -- | -- | -- |
| Trehalose | -- | -- | -- | -- | 80mM | 25mM | 25mM |
| Mannitol | -- | -- | 220mM | 220mM | 220mM | 220mM | 220mM |
| Sucrose | -- | | 25mM | 53mM | -- | -- | -- |

**Table 15. Lyophilization procedure of HAase**

| Operation steps | Set plate temperature (°C) | Holding time (hr) | Set vacuum level (mbar) |
|---|---|---|---|
| (1) Pre-freezing | -5 | 1.0 | / |
| (2) Pre-freezing | -40 | 3 | / |
| (3) Drying | -25 | 19.5 | 15 |
| (4) Drying | -20 | 1.5 | 15 |
| (5) Drying | 0 | 2.0 | 15 |
| (6) Drying | 25 | 8.5 | 15 |

### Example 12. Dry Meropenem powder preparation for subcutaneous administration

The lyophilized HAase powder was mixed with a dry Meropenem powder for injection, to prepare a dry Meropenem powder preparation for subcutaneous injection. The dry Meropenem powder for injection comprises sodium carbonate as a co-solvent. The specification of the mixture was that each portion comprises 1 g of Meropenem active ingredient, and 3,000 units, 10,000 units, 40,000 units or 120,000 units of HAase respectively according to different groups.

The mixed dry powder was dissolved in such a manner that the dry powder containing 1 g of Meropenem active ingredient was dissolved in 20 ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Meropenem content and appearance of samples at each sampling point were determined. The HAase activity and Meropenem content were detected by the methods described in "Chinese Pharmacopoeia".

Tables 16-17 show the changes of Meropenem content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Meropenem content is stable and the HAase activity is stable. At 1 hr, in the solutions of dry powder preparations having different compositions, the Meropenem content is in the range of 90-110%. The HAase activity in group A and group B decreases slightly, and in group C, group D, group E, group F and group G, the HAase activity is well retained.

**Table 16. Meropenem content**

| Group | Meropenem content (%) | |
|---|---|---|
| | 15min | 1hr |
| A1 | 98.8 | 98.6 |
| A2 | 112.1 | 111.8 |
| A3 | 98.0 | 98.3 |
| A4 | 99.4 | 100.0 |
| B1 | 103.1 | 102.1 |
| B2 | 100.7 | 101.4 |
| B3 | 96.3 | 97.0 |
| B4 | 97.9 | 97.9 |
| C1 | 99.8 | 99.1 |
| C2 | 100.6 | 100.4 |
| C3 | 101.8 | 101.4 |
| C4 | 98.8 | 98.4 |
| D1 | 97.2 | 96.0 |
| D2 | 97.9 | 97.6 |
| D3 | 99.1 | 98.9 |
| D4 | 99.9 | 98.6 |
| E1 | 98.2 | 98.5 |
| E2 | 103.2 | 104.8 |
| E3 | 97.3 | 98.3 |
| E4 | 103.0 | 102.2 |
| F1 | 103.0 | 102.6 |
| F2 | 104.9 | 104.0 |
| F3 | 96.8 | 97.8 |
| F4 | 98.3 | 98.4 |
| G1 | 100.3 | 99.6 |
| G2 | 99.9 | 98.8 |
| G3 | 98.3 | 99.1 |
| G4 | 98.8 | 98.6 |

**Table 17. HAase activity**

| Group | HAase activity (units/ml) | | |
|---|---|---|---|
| | Control | 15min | 1hr |
| A1 | 158 | 131 | 103 |
| A2 | 511 | 404 | 340 |
| A3 | 2028 | 1537 | 1022 |
| A4 | 6037 | 4064 | 3059 |
| B1 | 142 | 103 | 81 |
| B2 | 496 | 388 | 315 |
| B3 | 2197 | 1700 | 1189 |
| B4 | 6069 | 3945 | 2985 |
| C1 | 161 | 160 | 123 |
| C2 | 521 | 477 | 401 |
| C3 | 2011 | 1687 | 1209 |
| C4 | 5874 | 4419 | 3636 |
| D1 | 151 | 146 | 114 |
| D2 | 473 | 405 | 377 |
| D3 | 2029 | 1799 | 1241 |
| D4 | 6202 | 4712 | 4101 |
| E1 | 144 | 141 | 131 |
| E2 | 488 | 379 | 313 |
| E3 | 2182 | 1888 | 1906 |
| E4 | 6074 | 5235 | 4487 |
| F1 | 139 | 125 | 125 |
| F2 | 511 | 409 | 347 |
| F3 | 1777 | 1289 | 1410 |
| F4 | 5654 | 5514 | 5174 |
| G1 | 136 | 124 | 141 |
| G2 | 498 | 492 | 492 |
| G3 | 1983 | 2014 | 1977 |
| G4 | 6310 | 5954 | 6338 |

### Example 13. Dry Ceftriaxone powder preparation for subcutaneous injection

The lyophilized HAase powder (compositions A-G in Table 14) was mixed with a dry Ceftriaxone powder, to prepare a dry Ceftriaxone powder preparation for subcutaneous injection. The specification of the mixture was that each portion comprises 1 g of Ceftriaxone active ingredient, and 600 units, 2000 units, 8000 units or 24000 units of HAase respectively according to different groups.

The mixed dry powder was dissolved in such a manner that the dry powder containing 1 g Ceftriaxone active ingredient was dissolved in 4ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Ceftriaxone content and appearance of samples at each sampling point were determined. The HAase activity and Ceftriaxone content were detected by the methods described in "Chinese Pharmacopoeia".

Tables 18-19 show the changes of Ceftriaxone content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Ceftriaxone content is stable and the HAase activity is stable. At 1 hr, in the solutions of dry powder preparations having different compositions, the Ceftriaxone content is in the range of 90-110%. The HAase activity in group A and group B decreases slightly, and in group C, group D, group E, group F and group G, the HAase activity is well retained.

**Table 18. Ceftriaxone content**

| Group | Ceftriaxone content (%) | |
|---|---|---|
| | 15min | 1hr |
| A1 | 99.3 | 99.6 |
| A2 | 92.5 | 93.8 |
| A3 | 103.0 | 104.0 |
| A4 | 105.5 | 104.8 |
| B1 | 96.7 | 97.0 |
| B2 | 98.2 | 99.5 |
| B3 | 98.5 | 98.1 |
| B4 | 106.0 | 106.4 |
| C1 | 89.2 | 88.5 |
| C2 | 96.8 | 96.0 |
| C3 | 105.2 | 105.3 |
| C4 | 108.5 | 110.2 |
| D1 | 105.7 | 105.0 |
| D2 | 94.9 | 93.6 |
| D3 | 97.0 | 97.6 |
| D4 | 101.0 | 101.0 |
| E1 | 103.9 | 104.9 |
| E2 | 94.0 | 94.3 |
| E3 | 103.2 | 102.2 |
| E4 | 100.4 | 101.3 |
| F1 | 96.5 | 95.2 |
| F2 | 101.0 | 101.4 |
| F3 | 95.2 | 96.2 |
| F4 | 105.5 | 106.1 |
| G1 | 97.3 | 98.4 |
| G2 | 102.8 | 102.0 |
| G3 | 105.8 | 106.1 |
| G4 | 99.3 | 99.6 |

**Table 19. HAase activity**

| Group | HAase activity (units/ml) | | |
|---|---|---|---|
| | Control | 15min | 1hr |
| A1 | 156 | 117 | 95 |
| A2 | 495 | 367 | 315 |
| A3 | 2147 | 1764 | 1018 |
| A4 | 6294 | 4156 | 3362 |
| B1 | 152 | 115 | 106 |
| B2 | 516 | 406 | 334 |
| B3 | 1911 | 1554 | 800 |
| B4 | 6300 | 4523 | 3243 |
| C1 | 154 | 147 | 104 |
| C2 | 486 | 402 | 391 |
| C3 | 2186 | 1695 | 1498 |
| C4 | 6003 | 4443 | 4003 |
| D1 | 161 | 148 | 123 |
| D2 | 514 | 472 | 390 |
| D3 | 1810 | 1407 | 941 |
| D4 | 6237 | 5200 | 4556 |
| E1 | 160 | 141 | 153 |
| E2 | 478 | 366 | 283 |
| E3 | 2023 | 1780 | 1715 |
| E4 | 5939 | 4882 | 4542 |
| F1 | 169 | 168 | 149 |
| F2 | 486 | 391 | 311 |
| F3 | 2068 | 1690 | 1785 |
| F4 | 6273 | 5826 | 5779 |
| G1 | 152 | 148 | 148 |
| G2 | 482 | 479 | 475 |
| G3 | 1971 | 1964 | 1983 |
| G4 | 6066 | 5792 | 5988 |

### Example 14. Dry Azithromycin powder preparation for subcutaneous injection

The lyophilized HAase powder (compositions A-G in Table 14) was mixed with a dry Azithromycin powder for injection, to prepare a dry Meropenem powder preparation for subcutaneous injection. The dry Azithromycin powder for injection comprises anhydrous citric acid, sodium hydroxide or phosphoric acid, sodium hydroxide as a co-solvent. The specification of the mixture was that each portion comprises 1 g of Azithromycin active ingredient, and 750 units, 2500 units, 10000 units or 30000 units of HAase respectively according to different groups.

The mixed dry powder was dissolved in such a manner that the dry powder containing 0.5 g of Azithromycin active ingredient was dissolved in 5 ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Azithromycin content and appearance of samples at each sampling point were determined. The HAase activity and Azithromycin content were detected by the methods described in "Chinese Pharmacopoeia".

Tables 20-21 show the changes of Azithromycin content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Azithromycin content is stable and the HAase activity is stable. At 1 hr, in the solutions of dry powder preparations having different compositions, the Azithromycin content is in the range of 90-110%. The HAase activity in group A and group B decreases slightly, and in group C, group D, group E, group F and group G, the HAase activity is well retained.

**Table 20. Azithromycin content**

| Group | Azithromycin content (%) | |
|---|---|---|
| | 15min | 1hr |
| A1 | 103.0 | 105.0 |
| A2 | 105.2 | 104.7 |
| A3 | 101.7 | 102.5 |
| A4 | 105.2 | 105.3 |
| B1 | 109.4 | 109.5 |
| B2 | 102.8 | 102.6 |
| B3 | 99.4 | 101.0 |
| B4 | 88.6 | 87.5 |
| C1 | 96.4 | 95.5 |
| C2 | 109.1 | 109.1 |
| C3 | 99.6 | 98.0 |
| C4 | 99.6 | 99.9 |
| D1 | 99.3 | 100.2 |
| D2 | 105.1 | 104.8 |
| D3 | 99.6 | 100.2 |
| D4 | 99.5 | 99.8 |
| E1 | 96.3 | 97.0 |
| E2 | 89.8 | 91.0 |
| E3 | 100.4 | 100.9 |
| E4 | 106.2 | 106.6 |
| F1 | 100.8 | 101.2 |
| F2 | 94.6 | 95.8 |
| F3 | 106.1 | 106.8 |
| F4 | 98.5 | 97.3 |
| G1 | 98.0 | 97.4 |
| G2 | 97.6 | 98.4 |
| G3 | 95.5 | 95.5 |
| G4 | 103.0 | 105.0 |

**Table 21. HAase activity**

| Group | HAase activity (units/ml) | | |
|---|---|---|---|
| | Control | 15min | 1hr |
| A1 | 147 | 104 | 105 |
| A2 | 524 | 435 | 343 |
| A3 | 2017 | 1577 | 1050 |
| A4 | 5927 | 4264 | 2831 |
| B1 | 141 | 108 | 90 |
| B2 | 517 | 425 | 350 |
| B3 | 2133 | 1658 | 1224 |
| B4 | 6178 | 4192 | 3437 |
| C1 | 149 | 133 | 112 |
| C2 | 473 | 411 | 308 |
| C3 | 1976 | 1726 | 1347 |
| C4 | 6042 | 4550 | 4021 |
| D1 | 148 | 112 | 103 |
| D2 | 527 | 450 | 414 |
| D3 | 1871 | 1611 | 1285 |
| D4 | 6249 | 5080 | 4183 |
| E1 | 153 | 145 | 158 |
| E2 | 484 | 368 | 290 |
| E3 | 1978 | 1853 | 1591 |
| E4 | 6113 | 5005 | 4722 |
| F1 | 151 | 139 | 142 |
| F2 | 486 | 376 | 292 |
| F3 | 1921 | 1548 | 1593 |
| F4 | 6052 | 5928 | 5604 |
| G1 | 146 | 137 | 146 |
| G2 | 527 | 520 | 512 |
| G3 | 1949 | 1936 | 1993 |
| G4 | 5639 | 5729 | 5599 |

### Example 15. Dry Cefradine powder preparation for subcutaneous injection

The lyophilized HAase powder (compositions A-G in Table 14) was mixed with a dry Cefradine powder for injection, to prepare a dry Cefradine powder preparation for subcutaneous injection. The dry Cefradine powder for injection comprises arginine as a co-solvent. The specification of the mixture was that each portion comprises 1 g of Cefradine active ingredient, and 600 units, 2000 units, 8000 units or 24000 units of HAase respectively according to different groups.

The mixed dry powder was dissolved in such a manner that the dry powder containing 1 g Cefradine active ingredient was dissolved in 4ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Cefradine content and appearance of samples at each sampling point were determined. The HAase activity and Cefradine content were detected by the methods described in "Chinese Pharmacopoeia".

Tables 22-23 show the changes of Cefradine content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Cefradine content is stable and the HAase activity is stable. At 1 hr, in the solutions of dry powder preparations having different compositions, the Cefradine content is in the range of 90-110%. The HAase activity in group A and group B decreases slightly, and in group C, group D, group E, group F and group G, the HAase activity is well retained.

**Table 22. Cefradine content**

| Group | Cefradine content (%) | |
|---|---|---|
| | 15min | 1hr |
| A1 | 95.6 | 95.6 |
| A2 | 101.6 | 101.3 |
| A3 | 101.9 | 102.8 |
| A4 | 98.9 | 98.0 |
| B1 | 100.5 | 100.9 |
| B2 | 100.9 | 99.5 |
| B3 | 105.7 | 105.2 |
| B4 | 95.1 | 95.7 |
| C1 | 108.3 | 107.2 |
| C2 | 101.0 | 101.4 |
| C3 | 91.9 | 92.5 |
| C4 | 103.6 | 103.6 |
| D1 | 95.5 | 95.0 |
| D2 | 90.4 | 89.3 |
| D3 | 93.9 | 94.7 |
| D4 | 100.3 | 100.6 |
| E1 | 103.1 | 103.5 |
| E2 | 101.2 | 100.1 |
| E3 | 98.4 | 98.6 |
| E4 | 105.7 | 106.8 |
| F1 | 102.3 | 103.3 |
| F2 | 99.0 | 99.5 |
| F3 | 106.0 | 105.0 |
| F4 | 94.0 | 93.4 |
| G1 | 103.5 | 102.8 |
| G2 | 105.2 | 106.2 |
| G3 | 94.7 | 94.2 |
| G4 | 95.6 | 95.6 |

**Table 23. HAase activity**

| Group | HAase activity (units/ml) | | |
|---|---|---|---|
| | Control | 15min | 1hr |
| A1 | 148 | 115 | 98 |
| A2 | 515 | 414 | 341 |
| A3 | 2014 | 1468 | 953 |
| A4 | 5822 | 3863 | 2990 |
| B1 | 156 | 125 | 107 |
| B2 | 509 | 420 | 302 |
| B3 | 2011 | 1611 | 1119 |
| B4 | 6016 | 3773 | 3040 |
| C1 | 144 | 126 | 94 |
| C2 | 493 | 442 | 349 |
| C3 | 1971 | 1704 | 1334 |
| C4 | 5865 | 4180 | 3876 |
| D1 | 145 | 126 | 100 |
| D2 | 535 | 448 | 424 |
| D3 | 1803 | 1405 | 1156 |
| D4 | 5928 | 4478 | 4120 |
| E1 | 145 | 127 | 126 |
| E2 | 512 | 430 | 381 |
| E3 | 1946 | 1923 | 1741 |
| E4 | 6292 | 5621 | 5138 |
| F1 | 148 | 119 | 128 |
| F2 | 514 | 448 | 329 |
| F3 | 2118 | 1792 | 1829 |
| F4 | 6245 | 5395 | 5522 |
| G1 | 146 | 142 | 145 |
| G2 | 485 | 478 | 491 |
| G3 | 2104 | 2115 | 2132 |
| G4 | 6100 | 6106 | 6251 |

### Example 16. Dry Teicoplanin powder preparation for subcutaneous injection

The lyophilized HAase powder (compositions A-G in Table 14) was mixed with a dry Teicoplanin powder, to prepare a dry Teicoplanin powder preparation for subcutaneous injection. The specification of the mixture was that each portion comprises 1 g of Teicoplanin active ingredient, and 750 units, 2500 units, 10000 units or 30000 units of HAase respectively according to different groups.

The mixed dry powder was dissolved in such a manner that the dry powder containing 0.4 g Teicoplanin active ingredient was dissolved in 6 ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Teicoplanin content and appearance of samples at each sampling point were determined. The HAase activity and Teicoplanin content were detected by the methods described in "Chinese Pharmacopoeia".

Tables 24-25 show the changes of Teicoplanin content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Teicoplanin content is stable and the HAase activity is stable. At 1 hr, in the solutions of dry powder preparations having different compositions, the Teicoplanin content is in the range of 90-110%. The HAase activity in group A and group B decreases slightly, and in group C, group D, group E, group F and group G, the HAase activity is well retained.

**Table 24. Teicoplanin content**

| Group | Teicoplanin content (%) | |
|---|---|---|
| | 15 min | 1 hr |
| A1 | 102.1 | 100.4 |
| A2 | 93.3 | 92.1 |
| A3 | 97.8 | 95.4 |
| A4 | 95.7 | 94.8 |
| B1 | 100.3 | 100.5 |
| B2 | 97.5 | 96.8 |
| B3 | 97.7 | 96.8 |
| B4 | 94.0 | 93.7 |
| C1 | 109.1 | 107.2 |
| C2 | 100.5 | 100.8 |
| C3 | 98.8 | 96.6 |
| C4 | 100.6 | 100.6 |
| D1 | 95.2 | 93.8 |
| D2 | 95.9 | 95.5 |
| D3 | 106.6 | 106.2 |
| D4 | 101.6 | 100.9 |
| E1 | 100.9 | 99.4 |
| E2 | 93.6 | 92.1 |
| E3 | 105.5 | 105.1 |
| E4 | 93.8 | 92.5 |
| F1 | 106.6 | 105.7 |
| F2 | 95.8 | 93.7 |
| F3 | 91.3 | 89.9 |
| F4 | 104.8 | 103.1 |
| G1 | 104.2 | 102.9 |
| G2 | 102.5 | 101.0 |
| G3 | 99.0 | 97.7 |
| G4 | 99.9 | 98.9 |

**Table 25. HAase activity**

| Group | HAase activity (units/ml) | | |
|---|---|---|---|
| | Control | 15min | 1hr |
| A1 | 146 | 112 | 91 |
| A2 | 493 | 363 | 305 |
| A3 | 2142 | 1588 | 1204 |
| A4 | 5881 | 3554 | 2614 |
| B1 | 144 | 113 | 105 |
| B2 | 526 | 440 | 353 |
| B3 | 1985 | 1495 | 968 |
| B4 | 6003 | 4360 | 3430 |
| C1 | 162 | 166 | 113 |
| C2 | 514 | 449 | 389 |
| C3 | 1856 | 1584 | 1131 |
| C4 | 5842 | 4096 | 3831 |
| D1 | 148 | 132 | 95 |
| D2 | 507 | 430 | 388 |
| D3 | 1756 | 1498 | 1060 |
| D4 | 6301 | 4939 | 4281 |
| E1 | 157 | 138 | 139 |
| E2 | 502 | 402 | 303 |
| E3 | 1904 | 1845 | 1466 |
| E4 | 5980 | 4837 | 4174 |
| F1 | 142 | 131 | 120 |
| F2 | 498 | 378 | 314 |
| F3 | 1985 | 1446 | 1650 |
| F4 | 6065 | 5428 | 5469 |
| G1 | 153 | 165 | 176 |
| G2 | 502 | 501 | 482 |
| G3 | 2032 | 2004 | 2128 |
| G4 | 6164 | 6005 | 6248 |

### Example 17. Dry Ceftazidime powder preparation for subcutaneous injection

The lyophilized HAase powder (compositions A-G in Table 14) was mixed with a dry Ceftazidime powder, to prepare a dry Ceftazidime powder preparation for subcutaneous injection. The specification of the mixture was that each portion compris es 1 g of Ceftazidime active ingredient, and 750 units, 2500 units, 10000 units or 30000 units of HAase respectively according to different groups.

The mixed dry powder was dissolved in such a manner that the dry powder containing 1 g Ceftazidime active ingredient was dissolved in 5ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Ceftazidime content and appearance of samples at each sampling point were determined. The HAase activity and Ceftazidime content were detected by the methods described in "Chinese Pharmacopoeia".

Tables 26-27 show the changes of Ceftazidime content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Ceftazidime content is stable and the HAase activity is stable. At 1 hr, in the solutions of dry powder preparations having different compositions, the Ceftazidime content is in the range of 90-110%. The HAase activity in group A and group B decreases slightly, and in group C, group D, group E, group F and group G, the HAase activity is well retained.

**Table 26. Ceftazidime content**

| Group | Ceftazidime content (%) | |
|---|---|---|
| | 15min | 1hr |
| A1 | 98.0 | 96.0 |
| A2 | 101.5 | 99.7 |
| A3 | 97.9 | 97.4 |
| A4 | 105.9 | 105.4 |
| B1 | 104.1 | 104.5 |
| B2 | 93.6 | 93.9 |
| B3 | 99.2 | 99.2 |
| B4 | 101.5 | 100.4 |
| C1 | 103.3 | 101.4 |
| C2 | 97.7 | 95.3 |
| C3 | 114.6 | 114.1 |
| C4 | 99.9 | 99.0 |
| D1 | 99.2 | 98.6 |
| D2 | 89.4 | 87.8 |
| D3 | 100.3 | 98.8 |
| D4 | 99.3 | 97.4 |
| E1 | 97.1 | 95.7 |
| E2 | 105.3 | 102.9 |
| E3 | 102.7 | 101.4 |
| E4 | 109.0 | 107.7 |
| F1 | 99.5 | 99.0 |
| F2 | 110.5 | 109.8 |
| F3 | 95.8 | 95.0 |
| F4 | 101.6 | 100.8 |
| G1 | 99.9 | 98.7 |
| G2 | 99.2 | 98.9 |
| G3 | 99.9 | 99.8 |
| G4 | 101.6 | 99.9 |

**Table 27. HAase activity**

| Group | HAase activity (units/ml) | | |
|---|---|---|---|
| | Control | 15min | 1hr |
| A1 | 144 | 126 | 98 |
| A2 | 501 | 378 | 346 |
| A3 | 1845 | 1364 | 835 |
| A4 | 5811 | 3874 | 2596 |
| B1 | 133 | 92 | 91 |
| B2 | 519 | 387 | 351 |
| B3 | 2006 | 1651 | 1008 |
| B4 | 5887 | 4443 | 2605 |
| C1 | 135 | 120 | 83 |
| C2 | 496 | 453 | 354 |
| C3 | 2019 | 1822 | 1349 |
| C4 | 6151 | 4554 | 4605 |
| D1 | 151 | 133 | 121 |
| D2 | 496 | 459 | 409 |
| D3 | 1837 | 1476 | 1078 |
| D4 | 5781 | 4512 | 4099 |
| E1 | 138 | 134 | 116 |
| E2 | 509 | 386 | 316 |
| E3 | 2107 | 1842 | 1915 |
| E4 | 5957 | 5135 | 4404 |
| F1 | 142 | 144 | 130 |
| F2 | 489 | 373 | 315 |
| F3 | 1934 | 1465 | 1561 |
| F4 | 5778 | 5111 | 5268 |
| G1 | 131 | 135 | 126 |
| G2 | 475 | 470 | 456 |
| G3 | 2218 | 2245 | 2133 |
| G4 | 5913 | 5908 | 5865 |

### Example 18. Dry Ertapenem powder preparation for subcutaneous administration

The lyophilized HAase powder (compositions A-G in Table 14) was mixed with a dry Ertapenem powder, to prepare a dry Ertapenem powder preparation for subcutaneous injection. The specification of the mixture was that each portion comprises 1 g of Ertapenem active ingredient, and 500 units, 1667 units, 6667 units or 20000 units of HAase respectively according to different groups.

The mixed dry powder was dissolved in such a manner that the dry powder containing 1 g of Ertapenem active ingredient was dissolved in 3.2 ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Ertapenem content and appearance of samples at each sampling point were determined. The HAase activity and Ertapenem content were detected according to "Determination of Ertapenem for injection and related substance by HPLC".

Tables 28-29 show the changes of Ertapenem content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Ertapenem content is stable and the HAase activity is stable. At 1 hr, in the solutions of dry powder preparations having different compositions, the Ertapenem content is in the range of 90-110%. The HAase activity in group A and group B decreases slightly, and in group C, group D, group E, group F and group G, the HAase activity is well retained.

**Table 28. Ertapenem content**

| Group | Ertapenem content (%) | |
|---|---|---|
| | 15min | 1hr |
| A1 | 98.7 | 97.3 |
| A2 | 98.6 | 97.2 |
| A3 | 106.6 | 105.3 |
| A4 | 99.6 | 98.6 |
| B1 | 97.8 | 96.8 |
| B2 | 100.0 | 97.6 |
| B3 | 104.3 | 102.7 |
| B4 | 97.4 | 96.1 |
| C1 | 96.8 | 95.6 |
| C2 | 97.4 | 96.2 |
| C3 | 101.2 | 100.5 |
| C4 | 96.8 | 96.1 |
| D1 | 94.9 | 94.2 |
| D2 | 100.1 | 99.5 |
| D3 | 94.2 | 93.7 |
| D4 | 98.4 | 96.5 |
| E1 | 102.2 | 100.4 |
| E2 | 102.8 | 102.4 |
| E3 | 104.1 | 103.7 |
| E4 | 101.0 | 99.6 |
| F1 | 101.1 | 100.7 |
| F2 | 104.2 | 103.4 |
| F3 | 100.2 | 99.1 |
| F4 | 104.2 | 103.3 |
| G1 | 107.6 | 106.0 |
| G2 | 96.0 | 95.3 |
| G3 | 100.4 | 99.6 |
| G4 | 98.0 | 96.1 |

**Table 29. HAase activity**

| Group | HAase activity (units/ml) | | |
|---|---|---|---|
| | Control | 15min | 1hr |
| A1 | 154 | 131 | 92 |
| A2 | 500 | 375 | 328 |
| A3 | 2080 | 1699 | 1090 |
| A4 | 5936 | 4059 | 2538 |
| B1 | 139 | 118 | 67 |
| B2 | 489 | 360 | 272 |
| B3 | 1999 | 1740 | 1027 |
| B4 | 5952 | 3915 | 2710 |
| C1 | 164 | 144 | 133 |
| C2 | 499 | 402 | 396 |
| C3 | 1884 | 1493 | 1267 |
| C4 | 6108 | 4585 | 3968 |
| D1 | 151 | 134 | 101 |
| D2 | 475 | 409 | 352 |
| D3 | 2115 | 1670 | 1401 |
| D4 | 6077 | 4755 | 4017 |
| E1 | 156 | 140 | 142 |
| E2 | 523 | 435 | 332 |
| E3 | 1799 | 1514 | 1567 |
| E4 | 6082 | 5075 | 4751 |
| F1 | 145 | 120 | 139 |
| F2 | 477 | 406 | 323 |
| F3 | 1969 | 1553 | 1685 |
| F4 | 5859 | 5089 | 5223 |
| G1 | 145 | 159 | 142 |
| G2 | 530 | 544 | 522 |
| G3 | 2067 | 2077 | 2111 |
| G4 | 5954 | 5811 | 5606 |

### Example 19. Dry Polymyxin B powder preparation for subcutaneous injection

The lyophilized HAase powder (compositions A-G in Table 14) was mixed with a dry Polymyxin B powder, to prepare a dry Polymyxin B powder preparation for subcutaneous injection. The specification of the mixture was that each portion comprises 500,000 units of Polymyxin B active ingredient, and 300 units, 1000 units, 4000 units or 12000 units of HAase respectively according to different groups.

The mixed dry powder was dissolved in such a manner that the dry powder containing 500,000 units of Polymyxin B active ingredient was dissolved in 2 ml of water for injection. After dissolution, the concentrations of HAase were 150 units/ml, 500 units/ml, 2000 units/ml and 6000 units/ml, respectively. The solution was allowed to stand at 25°C for 15 min and 1 hr. The HAase activity, Polymyxin B content and appearance of samples at each sampling point were determined. The HAase activity and Polymyxin B content were detected by the methods described in "Chinese Pharmacopoeia".

Tables 30-31 show the changes of Polymyxin B content (90-110% is acceptable) and HAase activity in each group under various conditions.

The results show that the solutions of dry powder preparations having different compositions were clear and transparent after reconstitution. At 15 min, in the solutions of dry powder preparations having different compositions, the Polymyxin B content is stable and the HAase activity is stable. At 1 hr, in the solutions of dry powder preparations having different compositions, the Polymyxin B content is in the range of 90-110%. The HAase activity in group A and group B decreases slightly, and in group C, group D, group E, group F and group G, the HAase activity is well retained.

**Table 30. Polymyxin B content**

| Group | Polymyxin B content (%) | |
|---|---|---|
| | 15min | 1hr |
| A1 | 102.0 | 101.2 |
| A2 | 102.5 | 101.6 |
| A3 | 102.6 | 102.3 |
| A4 | 97.9 | 97.3 |
| B1 | 101.7 | 100.1 |
| B2 | 94.6 | 92.3 |
| B3 | 104.6 | 104.8 |
| B4 | 112.9 | 111.3 |
| C1 | 101.2 | 99.5 |
| C2 | 104.6 | 103.7 |
| C3 | 101.9 | 101.1 |
| C4 | 91.1 | 90.4 |
| D1 | 97.0 | 95.3 |
| D2 | 102.3 | 102.1 |
| D3 | 108.7 | 106.8 |
| D4 | 107.2 | 106.7 |
| E1 | 108.1 | 107.4 |
| E2 | 100.6 | 99.8 |
| E3 | 101.5 | 100.0 |
| E4 | 99.2 | 97.6 |
| F1 | 107.5 | 108.0 |
| F2 | 99.8 | 97.6 |
| F3 | 105.9 | 105.1 |
| F4 | 96.3 | 95.5 |
| G1 | 100.9 | 100.0 |
| G2 | 96.9 | 95.8 |
| G3 | 99.0 | 98.3 |
| G4 | 105.6 | 103.1 |

**Table 31. HAase activity**

| Group | HAase activity (units/ml) | | |
|---|---|---|---|
| | Control | 15 min | 1 hr |
| A1 | 143 | 94 | 89 |
| A2 | 519 | 406 | 351 |
| A3 | 1871 | 1426 | 735 |
| A4 | 5865 | 3916 | 2988 |
| B1 | 148 | 103 | 94 |
| B2 | 507 | 428 | 346 |
| B3 | 2015 | 1578 | 939 |
| B4 | 5864 | 3979 | 2547 |
| C1 | 144 | 134 | 107 |
| C2 | 478 | 412 | 377 |
| C3 | 2109 | 1796 | 1367 |
| C4 | 5941 | 4477 | 4043 |
| D1 | 168 | 160 | 131 |
| D2 | 488 | 423 | 395 |
| D3 | 2141 | 1794 | 1353 |
| D4 | 6263 | 4814 | 4356 |
| E1 | 153 | 149 | 148 |
| E2 | 496 | 390 | 304 |
| E3 | 2027 | 1878 | 1769 |
| E4 | 5995 | 4977 | 4391 |
| F1 | 156 | 140 | 133 |
| F2 | 505 | 413 | 360 |
| F3 | 2114 | 1728 | 1819 |
| F4 | 6403 | 5761 | 5562 |
| G1 | 138 | 129 | 136 |
| G2 | 507 | 504 | 493 |
| G3 | 1986 | 1951 | 2037 |
| G4 | 6305 | 6297 | 6330 |

### Example 20. Subcutaneous administration of antibiotics combined with recombinant human HAase

The difference in pharmacokinetics between subcutaneous administration of antibiotics combined with recombinant human HAase and traditional intravenous administration of antibiotics in mice was studied. Ceftriaxone, Ceftazidime, Ertapenem, and Teicoplanin were used as model antibiotics. A total of 48 ICR male mice were used in each antibiotic model, and divided into two groups, each having 24 mice. The two groups include a tail vein injection group, and a subcutaneous administration group of antibiotics combined with recombinant human HAase. The single dose was 100-200 mg/kg, and the drugs was respectively formulated and administered according to the scheme for each group. Blood samples were taken 5 min, 15 min, 30 min, 45 min, 1 hr, 2 hrs, 4 hrs and 8 hrs after administration. The concentration of each model antibiotic in the sample was detected by LC-MS and the pharmacokinetic parameters were calculated.

Table 32 shows the pharmacokinetics of each model antibiotic in mice after administration by tail vein injection, and subcutaneous administration in combination with recombinant human HAase.

The results show that compared with administration by intravenous injection, subcutaneous administration in combination with recombinant human HAase gives a longer Tₘₐₓ and a lower Cₘₐₓ, so that the antibiotics can act for a longer time and have better safety in the body, which is conducive to improving the efficacy of the antibiotics while ensuring the safety, so as to achieve a better therapeutic effect. Compared with the traditional subcutaneous administration, the subcutaneous administration in combination with recombinant human HAase provides a higher drug absorption efficiency, and is thus an optimum route of administration.

**Table 32. Pharmacokinetics of each model antibiotic in mice**

| **Group** | | **Tₘₐₓ (min)** | **Cₘₐₓ (mg/L)** | **AUCₗₐₛₜ(mg/L·h)** |
|---|---|---|---|---|
| Ceftriaxone | Administration by injection via tail vein | 5 | 384 | 406 |
| | Conventional subcutaneous administration | 15 | 324 | 473 |
| | In combination with recombinant human HAase for subcutaneous administration | 15 | 357 | 498 |
| Ceftazidime | Administration by injection via tail vein | 5 | 371 | 511 |
| | Conventional subcutaneous administration | 15 | 322 | 542 |
| | In combination with recombinant human HAase for subcutaneous administration | 15 | 331 | 558 |
| Ertapenem | Administration by injection via tail vein | 5 | 368 | 450 |
| | Conventional subcutaneous administration | 15 | 318 | 442 |
| | In combination with recombinant human HAase for subcutaneous administration | 15 | 333 | 459 |
| Teicoplanin | Administration by injection via tail vein | 5 | 148 | 195 |
| | Conventional subcutaneous administration | 15 | 121 | 201 |
| | In combination with recombinant human HAase for subcutaneous administration | 15 | 133 | 210 |

### Example 21. Advantages of intradermal or subcutaneous administration of antibiotics combined with recombinant human HAase

Ceftriaxone, Ceftazidime, Ertapenem, and Teicoplanin were used as model antibiotics. The effects of theses antibiotics orally administered alone and subcutaneously administered in combination with recombinant human HAase on intestinal microorganisms in mice were studied.

12 SPF grade C57BL/6 mice were used for each antibiotic mode, and divided into two groups, each having 6 mice. The two groups include an oral administration group, and a subcutaneous administration group combined with recombinant human HAase. A blank control group including 6 mice was also set. The mice in the oral administration group were given the model antibiotic solution by oral gavage for five consecutive days. The mice in the subcutaneous administration group combined with recombinant human HAase were given the same dose of model antibiotic as that in the oral administration group by subcutaneous administration for five consecutive days. The feces of mice in each group were collected on the 1st, 2nd, 3rd, 4th, 5th, 6th, 8th, 10th and 14th days after five consecutive days of administration, and stored at -80°C, for detecting the microbial diversity later. The fecal samples of each group were amplified and sequenced by 16S rDNA sequencing. After DNA extraction, PCR amplification and gene sequencing, the changes of intestinal bacterial diversity in each group of mice were analyzed. The relative species diversity of the test group was calculated, with the OUT value of the blank control group in each day as a reference, as shown in Table 33.

The results show that the change in diversity of intestinal flora in mice in the subcutaneous administration group combined with recombinant human HAase is less than that in mice in the oral administration group. The intestinal flora imbalance in mice in the subcutaneous administration group combined with recombinant human HAase is better than that in mice in the oral administration group. Compared with the traditional oral administration of antibiotics, the subcutaneous administration of antibiotics combined with recombinant human HAase can reduce the adverse effects on the intestinal microbial flora during treatment with antibiotics, so as to better protect the intestinal microbial diversity.

**Table 33. Effects of different administration routes on the diversity of intestinal flora in mice (expressed by OTU)**

| **Relative abundance of OUT (operational taxonomic units)** | | **D1** | **D2** | **D3** | **D4** | **D5** | **D6** | **D8** | **D10** | **D14** |
|---|---|---|---|---|---|---|---|---|---|---|
| Ceftriaxone | Oral administration | 16.8% | 14.7% | 10.4% | 14.1% | 15.5% | 18.3% | 15.8% | 23.0% | 25.7% |
| | In combination with recombinant human HAase for subcutaneous administration | 31.5% | 28.8% | 28.9% | 33.2% | 29.1% | 33.2% | 37.9% | 39.3% | 47.1% |
| Ceftazidime | Oral administration | 22.8% | 20.9% | 20.4% | 21.1% | 19.2% | 22.3% | 26.8% | 29.6% | 32.5% |
| | In combination with recombinant human HAase for subcutaneous administration | 35.5% | 34.0% | 34.3% | 33.7% | 34.3% | 35.1% | 41.6% | 42.3% | 51.3% |
| Cefradine | Oral administration | 24.4% | 23.6% | 20.4% | 22.6% | 20.7% | 24.3% | 25.8% | 28.6% | 31.9% |
| | In combination with recombinant human HAase for subcutaneous administration | 35.5% | 37.2% | 36.3% | 36.2% | 36.2% | 37.1% | 42.1% | 42.9% | 48.7% |
| Ertapenem | Oral administration | 16.2% | 13.1% | 10.4% | 14.6% | 16.0% | 15.3% | 15.8% | 20.9% | 24.1% |
| | In combination with recombinant human HAase for subcutaneous administration | 30.5% | 30.9% | 28.9% | 31.2% | 28.2% | 33.2% | 36.8% | 36.7% | 39.3% |
| Teicoplanin | Oral administration | 44.7% | 41.9% | 39.8% | 41.2% | 39.4% | 41.1% | 46.3% | 45.9% | 49.7% |
| | In combination with recombinant human HAase for subcutaneous administration | 51.8% | 56.0% | 53.7% | 56.3% | 51.2% | 55.4% | 63.7% | 64.8% | 71.7% |

Detailed methods of the present invention are described by way of examples above, but the present invention is not limited thereto. That is, the present invention can be implemented otherwise with no need to have to rely on the above-mentioned detailed methods. It is to be understood by those skilled in the art that any improvement made to the present invention, equivalent replacement of raw materials, addition of auxiliary components, and selection of specific methods, etc. fall within the scope of protection and disclosure of the present invention.

## Claims

1. A pharmaceutical composition for intradermal or subcutaneous administration, comprising an antibiotic and hyaluronidase (HAase).

2. The pharmaceutical composition according to claim 1, wherein the content of the antibiotic is 10 mg/mL-5 g/mL; and the content of the HAase is 45 units/ml-4500000 units/ml.

3. The pharmaceutical composition according to claim 1 or 2, wherein the antibiotic is selected from the group consisting of β-lactams, aminoglycosides, macrolides, lincomycins, polypeptides, tetracyclines, quinolones, sulfanilamides, anti-tuberculosis agents, and antifungal agents.

4. The pharmaceutical composition according to claim 3, wherein the β-lactam antibiotics are selected from the group consisting of Penicillin G, Penicillin V, Pheneticillin, Oxacillin, Methicillin, Cloxacillin, Dicloxacillin, Ampicillin, Amoxicillin, Pivampicillin, Carbenicillin, Piperacillin, Sulbenicillin, Temocillin, Mezlocillin, Mecillinam, Amdinocillin Pivoxil, Apalcillin, Aspoxicillin, Azidocillin, Azlocillin, Bacampicillin, Benzylpenicillin, Benzylpenicillin sodium, Carindacillin, Clometocillin, Ciclacillin, Epicillin, Fenbenicillin, Flucloxacillin, Hetacillin, Lenampicillin, Metampicillin, Methicillin sodium, Nafcillin, Penamecillin, Penethamate hydriodide, Penicillin G Benethamine, Penicillin G Benzathine, Penicillin G benzhydrylamine, Penicillin G calcium, Penicillin G hydrabamine, Penicillin G potassium, Penicillin G procaine, Penicillin N, Penicillin O, Penicillin V Benzathine, Penicillin V hydrabamine, Penimepicycline, Pheneticillin potassium, Propicillin, Quinacillin, Sulbenicillin, Sultamicillin, Talampicillin, Tazocillin, Ticarcillin, Loracarbef, 3-chloro-1-carbacephem, 3-thiocarbacephem, Flomoxef, Latamoxef, Latamoxef, Cefazolin, Cefalexin, Cefalotin, Cefradine, Ceftezole, Cefuroxime, Cefaclor, Cefamandole, Cefotiam, Cefonicid, Ceforanide, Cefoperazone, Ceftriaxone, Ceftazidime, Cefotaxime, Ceftizoxime, Cefixime, Cefodizime, Cefpiramide, Cefpirome, Cefepime, Cefclidin, Cefadroxil, Cefatrizine, Cefazedone, Cefcapene pivoxil, Cefolidin, Cefdinir, Cefditoren, Cefetamet, Cefmenoxime, Cefotetan, Cefozopran, Cefpimizole, Cefpodoxime Proxetil, Cefprozil, Cefroxadine, Cefsulodin, Cefteram, Ceftibuten, Ceftizoxime, Cefuzonam, Cephatril, Cefaloglycin, Cefaloridine, Cephalosporin, Cephalotin, Cephapirin, Cefbuperazone, Cefminox, Imipenem, Meropenem, Panipenem, Biapenem, Ertapenem, Faropenem, Cefoxitin, Cefmetazole, Aztreonam, Carumonam, Latamoxef, Flomoxef, clavulanic acid, clavulanate, clavulanate, Sulbactam, and Tazobactam;
the aminoglycoside antibiotics are selected from the group consisting of Streptomycin, Kanamycin, Gentamicin, Amikacin, Tobramycin, Netilmicin, Sisomicin, Albomycin, Arbekacin, Bambermycin, Butyrosin, Dibekacin, Dihydrostreptomycin, Fortimicin, Isepamicin, Micronomicin, Neomycin, Neodecyllin, Paromomycin, Ribostamycin, Spectinomycin, and Trospectomycin;
the macrolide antibiotics are selected from the group consisting of Erythromycin, Clarithromycin, Azithromycin, Roxithromycin, Carbomycin, Dirithromycin, Erythromycin Acistrate, Erythromycin Estolate, Erythromycin Gluceptate, Erythromycin Lactobionate, Erythromycin Stinoprate, Erythromycin stearate, Josamycin, Leucomycin, Midecamycin, Miokamycin, Oleandomycin, Primycin, Rokitamycin, Rosaramicin, Spiramycin, and Troleandomycin;
the lincomycin antibiotics are selected from the group consisting of Lincomycin and Clindamycin;
the polypeptide antibiotics are selected from the group consisting of Polymixin B, Polymixin E, Norvancomycin, Teicomycin, Vancomycin, Teicoplanin, Amphomycin, Bacitracin, Capreomycin, Colistin, Colomycin, Enduracidin, Enviomycin, Fusafungine, Gramicidin, Mikamycin, Polymixin, Pristinamycin, Dalfopristin, Ristocetin, Thiostrepton, Tuberactinomycin, Tyrocidine, Tyrothricina, Viomycin, Virginiamycin, and Zinc Bacitracin;
the tetracycline antibiotics are selected from the group consisting of Apicycline, Chlortetracycline, Clomocycline, Demeclocycline, Doxycycline, Guamecycline, Lymecycline, Meclocycline, Metacycline, Minocycline, Oxytetracycline, Penimepicycline, Pipacycline, Rolitetracycline, Sancycline, Tetracycline, Cycloserine, Mupirocin, and Tuberin;
the quinolone antibiotics are selected from the group consisting of Nalidixic Acid, Pipemidic Acid, Norfloxacin, Ofloxacin, Ciprofloxacin, Enoxacin, Pefloxacin, Levofloxacin, Gatifloxacin, Moxifloxacin, Norfloxacin, Fleroxacin, Lomefloxacin, Sparfloxacin, Grepafloxacin, Rufloxacin, Clinafloxacin, Balofloxacin, Trovafloxacin, Fluoroquinolone, Alatrofloxacin Mesylate, Cinoxacin, Difloxacin, Flumequine, Grepafloxacin, Miloxacin, Marbofloxacin, Nadifloxacin, Oxolinic acid, Pazufloxacin, Piromidic acid, Rosoxacin, Temafloxacin, Tosufloxacin, and Trovafloxacin mesilate;
the sulfanilamide antibiotics are selected from the group consisting of Sulfamethoxazole, Trimethoprim, acetyl Sulfamethoxypyridazine, Benzylsulfamide, Chloramine B, Chloramine T, Dichloramine T, Sulfisomidine, β-Glucosyl Sulfanilamide, Mafenide, 4'-methylamino sulfonyl-sulfonanilide, Noprylsulfamide, Phthalylsulfacetamide, Phthalylsulfathiazole, Salicylazosulfapyridine, Sulfasuxidine, Sulfabenzamide, Sulfacetamide, Sulfachlorpyridazine, Sulfachrysoidine, Sulfacitine, Sulfadiazine, Sulfadicramide, Sulfadimethoxine, Sulfadoxine, Sulfaethidole, Sulfaguanidine, Sulfaguanole, Sulfalene, Sulfaloxic Acid, Sulfamerazine, Oxymethylpyrimidine, Sulfamerazine, Sulfamethizole, Sulfametomidine, Sulfisoxazole, Sulfamethoxypyridazine, Sulfametrole, Sulfamidochrysoidine, Sulfazole, Sulfanilamide, 4-Sulfanilamidosalicylic acid, Sulfanilamidosulfanilamide, Sulfanilylurea, n-Sulfanilamido-3,4-dicarboxamide, Sulfanitran, Sulfaperin, Sulfaphenazole, Sulfaproxyline, Sulfapyrazole, Sulfapyridine, Sulfasomizole, Sulfasymazine, Sulfathiazole, Sulfathiourea, Sulfatolamide, Sulfisomidine, and Sulfatroxazole;
the furan antibiotics are selected from the group consisting of Nitrofurantoin, Furazolidone, Furaltadone, Furazolium Chloride, Nifuradene, Nifuratel, Nifurfoline, Nifurpirinol, Nifurprazine, and Nifurtoinol;
the nitroimidazole antibiotics are selected from the group consisting of Metronidazole, Tinidazole and Omidazole;
the anti-tuberculosis antibiotics is selected from the group consisting of Isoniazid, Rifampicin, Rifamide, Pyrazinamide and Ethambutol; and/or
the antifungal antibiotics are selected from the group consisting of amphotericin B, fluconazole, Itraconazole, 5-Flucytosine, Candicidin, Dermostatin, Filipin, antifungal pigments, Hachimycin, Hamycin, Lucensomycin, Mepartricin, Pimaricin, Natamycin, Nysfungin, Pecilocin, Permycin, Azaserine, Griseofulvin, Oligomycin, Neodecyllin, Pyrrolnitrin, Siccanin, Tubercidin, Viridin, Allyl amine, Butenafine, Naftifine, Terbinafine, imidazole, Bifonazole, Butoconazole, Chlordantoin, Chlormidazole, Cloconazole, Clotrimazole, Econazole, Enilconazole, Fenticonazole, Flutrimazole, Isoconazole, Ketoconazole, Lanoconazole, Miconazole, Omoconazole, Oxiconazole nitrate, Sertaconazole, Sulconazole, Tioconazole, Voriconazole, thiocarbamate, Tolciclate, Tolindate, Tolnaftate, triazole, Saperconazole, Terconazole, Acrisorcin, Amorolfine, Xenysalate, Bromosalicylchloranilide, Buclosamide, Calcium propionate, Chlorphenesin, Coparaffinate, Dimazole dihydrochloride, Exalamide, Flucytosine, Haletazole, Hexetidine, lipopeptide such as Echinocandin, Loflucarban, Nifuratel, Potassium iodide, Propanoic acid, 2-Mercaptopyridine oxide, Salicylanilide, Sodium propionate, Sulbentine, Tenonitrozole, Triacetin, Benzthiadiazine acetate, Undecenoic acid, and Zinc propionate.

5. The pharmaceutical composition according to any one of claims 1 to 4, wherein the HAase has the activity of degrading hyaluronic acid under neutral conditions; preferably, the HAase is selected from the group consisting of HAase extracted from animal testis, recombinant animal HAase or a mutant thereof, recombinant and/or extracted bacterial HAase, recombinant human HAase or a mutant thereof; preferably, the HAase is recombinant human HAase or a mutant thereof; further preferably, the HAase is recombinant human HAase or a mutant thereof; more preferably, the recombinant human HAase comprises an amino acid sequence as shown in SEQ ID NO. 1; and most preferably, the recombinant human HAase comprises an amino acid sequence as shown in SEQ ID NO. 2.

6. The pharmaceutical composition according to any one of claims 1 to 5, wherein the HAase has an enzyme activity of 45 units/ml-3000000 units/ml, preferably 45 units/ml-1500000 units/ml, more preferably 50 units/ml-30000 units/ml, and most preferably 100 units/ml-3000 units/ml.

7. The pharmaceutical composition according to any one of claims 1 to 6, wherein the Cₘₐₓ of the antibiotic in the composition is increased by at least 10%, for example, at least 20%, at least 30%, at least 40%, and at least 50%, compared with that in the case where the antibiotic is administered alone; and/or the Tₘₐₓ of the antibiotic in the composition is reduced by at least 20%, for example, at least 30%, at least 40%, and at least 50%, compared with that in the case where the antibiotic is administered alone; and/or the AUCₗₐₛₜ of the antibiotic in the composition is increased by at least 5%, or at least 10%, compared with that in the case where the antibiotic is administered alone.

8. The pharmaceutical composition according to any one of claims 1 to 7, wherein when the composition is subcutaneously administered, the reduction in the diversity of intestinal microbial flora relative to that before administration is 90% or less, for example, 80% or less, 70% or less, 60% or less, and 50% or less, of the reduction relative to the diversity before administration when the antibiotic is administered orally.

9. The pharmaceutical composition according to any one of claims 1 to 8, optionally comprising a pharmaceutically acceptable adjuvant.

10. The pharmaceutical composition according to claim 9, wherein the pharmaceutically acceptable adjuvant is selected from the group consisting of a buffer, a stabilizer, a nonionic surfactant, a co-solvent, a preservative and/or an excipient.

11. The pharmaceutical composition according to claim 10, wherein the buffer is selected from the group consisting of a histidine buffer, an acetic acid buffer, a phosphate buffer, a citric acid buffer, and Tris buffer, preferably, a phosphate buffer, and most preferably disodium hydrogen phosphate;
the stabilizer is selected from the group consisting of trehalose, sucrose, mannitol, sodium chloride, methionine, and disodium edetate, preferably trehalose, mannitol, methionine, and sucrose, and most preferably, a combination of methionine and trehalose;
the nonionic surfactant is selected from the group consisting of polysorbate 20, polysorbate 80, and poloxamer 188, preferably polysorbate 20 and polysorbate 80, and most preferably polysorbate 20;
the co-solvent is selected from the group consisting of sodium carbonate, phosphoric acid, citric acid, sodium bicarbonate, sodium hydroxide, sodium chloride, and L-arginine, and preferably sodium bicarbonate and sodium hydroxide;
the preserver is selected from the group consisting of glycerol, methyl paraben, propyl paraben, benzoic acid, sodium benzoate, and ethanol; and/or
the excipient is selected from the group consisting of sorbitol, mannitol, trehalose, glycerol, lactose, sucrose, trehalose, maltose and glucose, preferably mannitol, trehalose and sucrose, and most preferably a combination of trehalose and mannitol.

12. The pharmaceutical composition according to claim 11, wherein
the concentration of the buffer is 1-100 mM, and preferably 5-50 mM, for example, 5 mM, 10 mM or 50 mM;
the concentration of the stabilizer is 1-500 mM, and preferably 30-150 mM, for example, 5mM methionine+25mM trehalose, 5mM methionine+53mM trehalose, 5mM methionine+100mM trehalose, lOmM methionine+25mM trehalose, lOmM methionine+53mM trehalose, lOmM methionine+100mM trehalose, 50mM methionine+25mM trehalose, 50mM methionine+53mM trehalose, and 50 mM methionine+100mM trehalose;
the concentration of the excipient is 1-500 mM, and preferably 160-280 mM, for example, 160 mM, 220 mM or 280 mM;
the concentration of the surfactant is 0.01-0.1% (w/v), preferably 0.02-0.04% (w/v), and most preferably 0.02% (w/v); and/or
the concentration of the co-solvent is 0.01 g/L-100 g/L.

13. The pharmaceutical composition according to any one of claims 1 to 12, which is a lyophilized preparation and/or a liquid preparation.

14. A kit, comprising the pharmaceutical composition according to any one of claims 1 to 13, wherein the antibiotic and the hyaluronidase (HAase) in the kit are packaged in mixture or separately.

15. The kit according to claim 14, wherein the separately packaged antibiotic and/or the separately packaged HAase are lyophilized preparations or liquid preparations.

16. The kit according to claim 15, wherein the lyophilized preparation is reconstituted before administration to a subject, and the liquid preparation is directly administered to the subject or diluted before administration to the subject.

17. The kit according to any one of claims 14 to 16, wherein the antibiotic and the HAase are administered sequentially or simultaneously, and the separately packaged antibiotic and/or the separately packaged HAase are administered sequentially or simultaneously.

18. The kit according to claim 17, wherein the antibiotic and the HAase are administered sequentially through a three-way infusion tube, and the administration speed is preferably controlled by manually pushing, by an infusion pump, or by gravity.

19. A method for preparing the kit according to any one of claims 15 to 18, comprising:
(a) providing an antibiotic;
(b) providing hyaluronidase (HAase); and
(c) optionally providing a pharmaceutically acceptable adjuvant,
wherein the antibiotic and the HAase are respectively prepared into a lyophilized preparation or a liquid preparation; or the antibiotic and the HAase are mixed and then prepared into a lyophilized preparation or a liquid preparation.

20. An injection system, selected from the group consisting of a syringe, an infusion pump, an injection pen, a needleless device, and other delivery devices, wherein the injection system is filled with the pharmaceutical composition according to any one of claims 1 to 14.

21. Use of the pharmaceutical composition according to any one of claims 1 to 14 and the kit according to any one of claims 15 to 18 in the preparation of drugs for treating diseases selected from the group consisting of infections with bacteria, fungi, actinomycetes, mycoplasma, chlamydia, spirochete, and amoeba and diseases caused by the infections.

22. The use according to claim 21, wherein the bacteria are selected from the group consisting of Escherichia, Pseudomonas, Klebsiella, Acinetobacter, Enterobacter, Citrobacter, Haemophilus, Proteus, Salmonella, Shigella, Serratia, Corynebacterium, Staphylococcus, Streptococcus, Enterococcus, Neisseria, Mycobacterium, and Legionella; and
the fungi are selected from the group consisting of Trichophyton, Epidermophyton, Microsporum, Candida, Cryptococcus, Coccidioides, Histoplasma, Sporothrix, Blastomyces, Geotrichum, Aspergillus, Mucor and Penicillium.

23. The use according to claim 21 or 22, wherein the bacteria or fungi are selected from the group consisting of Haemophilus influenzae type B, Pseudomonas aeruginosa types A and B, Staphylococcus aureus, Streptococcus group B, Streptococcus pneumoniae types (1, 3, 4, 6, 7, 8, 9, 12, 14, 18, 19 and 23), *Staphylococcus epidermidis*, *Staphylococcus saprophyticus*, *Streptococcus pyogenes, Streptococcus viridans, Streptococcus agalactiae, Diplococcus pneumoniae, Bacillus anthracis, Corynebacterium diphtheriae, Bordetella pertussis, Bacillus tetani, Mycobacterium tuberculosis, Bacillus, Clostridium perfringens, Mycobacterium leprae, Gonococcus, Meningococcus, Legionella pneumophila, Shigella dysenteriae, Pseudomonas aeruginosa, Bacillus proteus, Vibrio cholerae, Vibrio parahaemolyticus, Escherichia coli, Salmonella typhi, Salmonella paratyphi*, *Salmonella*, *Haemophilus influenzae*, *Acinetobacter*, *Aspergillus*, *Candida*, *Cryptococcus*, *Mucor* or *Fusarium.*

24. The use according to any one of claims 21 to 23, wherein the disease is chronic osteomyelitis caused by Staphylococcus aureus, endocarditis caused by drug-resistant Staphylococcus aureus or Enterococcus, typhoid fever, paratyphoid fever, bacterial food poisoning, bacterial infectious diarrhea, cholera, bacillary dysentery, brucellosis, plague, anthrax, diphtheria, pertussis, scarlatina, epidemic cerebrospinal meningitis, tuberculosis, bacterial blood infection, bacterial upper respiratory tract infection, bacterial lower respiratory tract infection, bacterial urinary tract infection, bacterial abdominal cavity infection, dermatophytosis, mycotic stomatitis, candida vaginitis, fungal pneumonia, fungal urinary tract infection, fungemia, cryptococcosis, candidiasis, aspergillosis, and pneumocystis.
